# EUROPEAN PATENT APPLICATION

(11) **EP 4 335 871 A1**
(43) Date of publication of application: **13.03.2024**
(21) Application number: 22799133.8
(22) Date of filing: 04.05.2022
(51) Int. Cl.: C07K 16/28, A61P 35/00, A61K 39/00

(54) **TIE2 AGONISTIC ANTIBODIES AND USES THEREOF**

(30) Priority: 06.05.2021 US 202163184901 P; 16.07.2021 KR 20210093451
(71) Applicant: Institute for Basic Science, Yuseong-gu Daejeon 34126 (KR); Korea Advanced Institute of Science and Technology, Daejeon 34141 (KR)
(72) Inventor: KIM, Ho Min, Daejeon 34141 (KR); KOH, Gou Young, Daejeon 34141 (KR); JO, Gyunghee, Daejeon 34141 (KR); BAE, Jeomil, Daejeon 34141 (KR)
(74) Representative: Mewburn Ellis LLP
(86) International application number: PCT/KR2022/006431
(87) International publication number: WO 2022/235090

(57) **Abstract**

The present invention relates to a Tie2 agonistic antibody that binds to an Ig3 Fn3 domain of human Tie2 or an antigen-binding fragment thereof, wherein by binding of the antibody, homodimer Tie2 can form a polygonal assembly so as to be clustered and activated.

## Description

### [Technical Field]

The present invention relates to a Tie2-agonistic antibody or antigen-binding fragment thereof binding to the Ig3 Fn3 (membrane proximal fibronectin type III) domain of human Tie2, in which, by binding of the antibody, homodimeric Tie2 may be formed into a polygonal assembly, and thus clustered and activated.

### [Background Art]

Angiogenesis occurs dynamically by various regulatory factors during development, growth, preservation, and homeostasis of an organism. As such, newly formed blood vessels serve as transport channels for various biomaterials, such as nutrients, oxygen, hormones, and the like, to surrounding cells. Functionally and structurally abnormal blood vessels are a direct or indirect cause of the onset and progression of various diseases. Tumor blood vessels worsen hypoxia due to functional and structural defects, resulting in tumor progression and metastasis to other tissues, and prevent anticancer drugs from being delivered well to the center of tumor tissue. In addition to cancer, defective blood vessels may be identified in various other diseases or disorders. Examples thereof include various eye diseases (e.g., diabetic macular edema, age-related macular degeneration), viral infections, and acute inflammatory responses such as sepsis, etc. Therefore, if a therapeutic agent that is able to normalize pathological blood vessels is present, application thereof to the treatment of various patients with vascular abnormalities is possible.

In order to inhibit abnormal angiogenesis and reduce vascular permeability, methods of directly activating Tie2 are being considered. Recombinant proteins that directly bind to the Tie2 receptor and induce Tie2 phosphorylation and activation are also being developed, and therapeutic effects thereof are being tested in a number of preclinical cancer and eye models. COMP-Ang1 and Vasculotide are representative examples. Although these agents exhibit anti-angiogenic and anti-invasive activities, they have the disadvantage of a very short half-life and unstable physicochemical properties. Moreover, a small molecule compound (AKB-9778) has been developed as an inhibitor of the dephosphorylating enzyme VE-PTP. VE-PTP serves to inactivate Tie2 by removing the phosphate group from phosphorylated Tie2. These compounds have the disadvantage of nonspecifically activating other receptors, but they indirectly increase Tie2 activity by inhibiting VE-PTP. Also, Tie2-activating antibodies have been developed (US6365154B1, US20170174789A1). These antibodies inhibit vascular leakage by increasing the survival rate of vascular endothelial cells. Interestingly, it is claimed that one of plant extracts may induce Tie2 activity and may be used as a skin care cosmetic (e.g., JP2011102273A, JP2018043949A, JP2015168656A).

Tie2 is an endothelial cell-specific receptor tyrosine kinase that promotes the growth and stability of blood vessels, and may be an attractive therapeutic target for ischemic and inflammatory vascular diseases. Tie2-agonistic antibodies and oligomeric angiopoietin (Angpt1) variants have been developed as potential therapeutic agents. However, the underlying mechanism for the role thereof in Tie2 clustering and activation has not been clearly identified. Moreover, Angpt1 variants have difficulties in production and storage.

Against this background, the inventors of the present application have made efforts to develop a Tie2-agonistic antibody, and thus developed a human Tie2-agonistic antibody and ascertained that such an antibody binds specifically to the Fn3 domain of Tie2, so that homodimeric Tie2 may be formed into a polygonal assembly and thus clustered and activated, thereby culminating in the present invention.

### [Disclosure]

It is an object of the present invention to provide a Tie2-agonistic antibody or antigen-binding fragment thereof.

It is another object of the present invention to provide a nucleic acid encoding the antibody or antigen-binding fragment thereof.

It is still another object of the present invention to provide a vector including the nucleic acid, cells transformed with the vector, and a method of producing the same.

It is yet another object of the present invention to provide a composition for preventing or treating an angiogenic disease including the antibody or antigen-binding fragment thereof.

It is still yet another object of the present invention to provide a composition for co-administration with an additional therapeutic agent for an angiogenic disease, including the antibody or antigen-binding fragment thereof.

In order to accomplish the above objects, the present invention provides a Tie2-agonistic antibody or antigen-binding fragment thereof, in which the antibody binds to the Ig3 Fn3 (membrane proximal fibronectin type III) domain of human Tie2, and by binding of the antibody, homodimeric Tie2 is formed into a polygonal assembly, and thus clustered and activated.

Particularly, the present invention provides Fab including a heavy-chain variable region (VH) including the sequence of SEQ ID NO: 1, a heavy-chain constant region (CH) including the sequence of SEQ ID NO: 3, a light-chain variable region (VL) including the sequence of SEQ ID NO: 2, and a light-chain constant region (CL) including the sequence of SEQ ID NO:4.

Particularly, the present invention provides a humanized antibody including a heavy-chain variable region including the amino acid sequence of SEQ ID NO: 5 or SEQ ID NO: 7 and a light-chain variable region including the amino acid sequence of SEQ ID NO: 6 or SEQ ID NO: 8.

In addition, the present invention provides a nucleic acid encoding the antibody or antigen-binding fragment thereof.

In addition, the present invention provides a vector including the nucleic acid.

In addition, the present invention provides cells transformed with the vector.

In addition, the present invention provides a method of producing the antibody or antigen-binding fragment thereof, including (a) culturing the cells and (b) recovering the antibody or antigen-binding fragment thereof from the cultured cells.

In addition, the present invention provides a composition for preventing or treating an angiogenesis-related disease, including the antibody or antigen-binding fragment thereof as an active ingredient.

In addition, the present invention provides a composition for co-administration with an additional therapeutic agent for an angiogenic disease, including the antibody or antigen-binding fragment thereof. The present invention provides a method of preventing or treating an angiogenic disease, including administering the antibody or antigen-binding fragment thereof to a patient. The present invention provides the use of the antibody or antigen-binding fragment thereof for the manufacture of a medicament for preventing or treating an angiogenic disease.

In addition, the present invention provides an antibody polygonal assembly including a Tie2-agonistic antibody or antigen-binding fragment thereof and formed with homodimeric Tie2 by binding of the antibody to the Ig3 Fn3 (membrane proximal fibronectin type III) domain of human Tie2.

### [Description of Drawings]

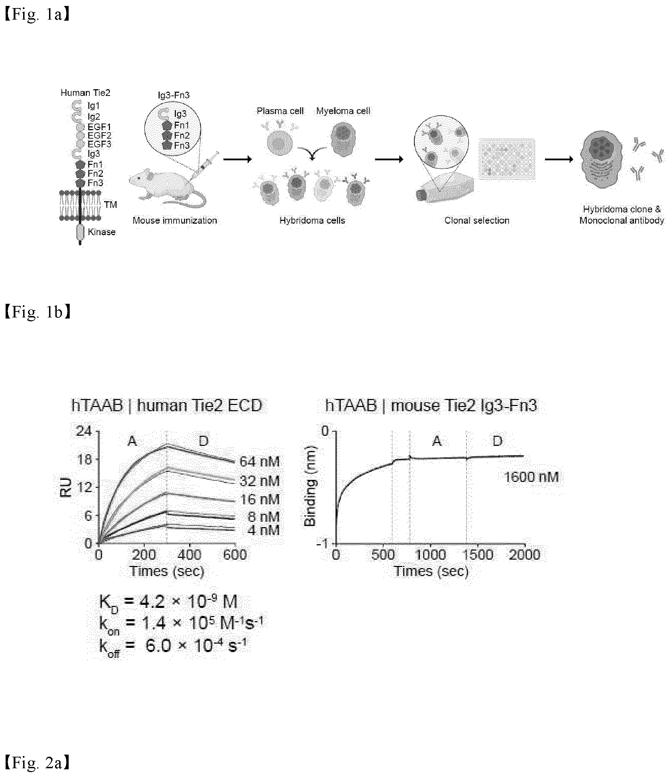

FIGs. 1a and 1b show production of a Tie2-activating mouse monoclonal antibody,
   a showing a schematic domain structure of the Tie2 receptor and a strategy for monoclonal antibody production by hybridoma technology, Ig: immunoglobulin-like domain, EGF: epidermal growth factor-like domain, Fn: fibronectin type III domain, and
   b showing binding kinetics of hTAAB for human (left) or mouse (right) Tie2 determined by SPR and BLI analysis, in which the equilibrium dissociation constant (K_{D}, M) was calculated as the ratio of off-rate to on-rate (k_{off}/kₒₙ), and kinetic parameters were determined with the global fitting function of Biacore Insight Evaluation Software using a 1:1-binding model;

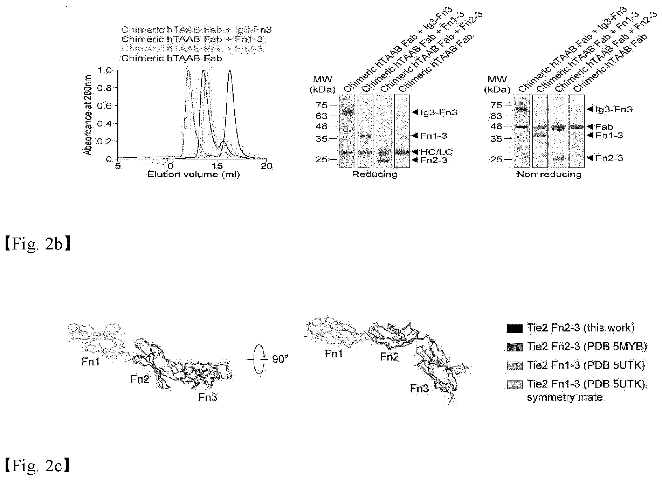

FIGs. 2a to 2d show results of structural comparison of Tie2 Fn2-3,
   a showing results of size-exclusion chromatography of chimeric hTAAB Fab alone (black line) and in complex with Tie2 ECD variants (Ig3-Fn3, magenta line; Fn1-3, blue line; and Fn2-3, yellow line) (left), and of analysis of elution fractions by SDS-PAGE and Coomassie blue staining under reducing and non-reducing conditions (right),
   b showing a comparison of the chimeric hTAAB Fab-bound Tie2 Fn2-3 monomer (black) with the previously reported monomer structures of Tie2 Fn2-3 (red, PDB: 5MYB chain A) and Fn1-3 (green, PDB: 5UTK chain A), in which the structures are represented as ribbon diagrams,
   c showing a comparison of the chimeric hTAAB Fab-bound Tie2 Fn2-3 dimer (black) with the previously reported Tie2 Fn2-3 dimer structure (red, PDB: 5MYB), and
   d showing a comparison of the chimeric hTAAB Fab-bound Tie2 Fn2-3 dimer (black) with the previously reported Tie2 Fn1-3 dimer structure (PDB: 5UTK), in which the Tie2 Fn1-3 dimers in two asymmetric units in the crystal lattice are colored in green and cyan, respectively;

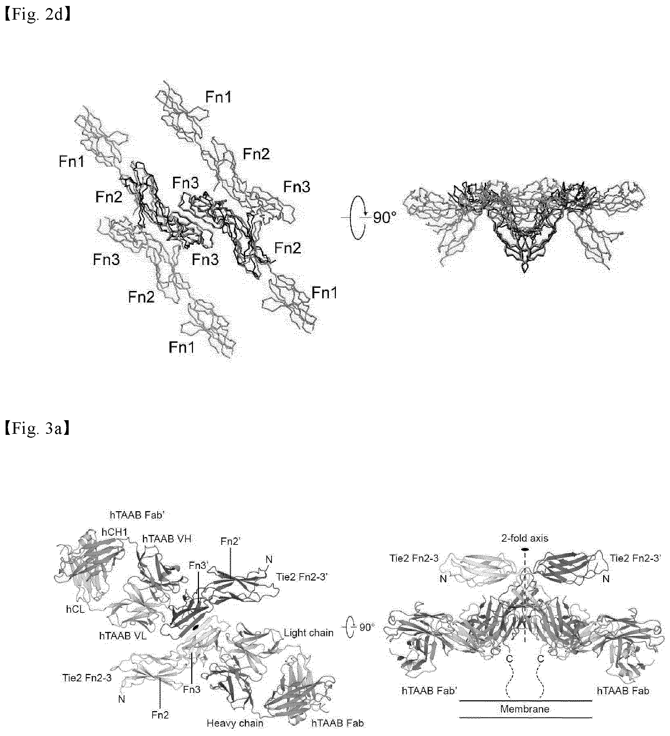

FIGs. 3a to 3d show the overall structure of hTAAB Fab in complex with Tie2 Fn2-3,
   a showing the overall structure of a 2:2 hTie2 Fn2-3/chimeric hTAAB Fab complex, in which the 2-fold axis of the dimeric complex is represented as a black ellipse, the heavy and light chains of chimeric hTAAB Fab are colored in dark orange and light orange, respectively, and the hTie2 Fn2-3 dimer is colored in teal and cyan,
   b showing a sequence comparison of human and mouse Tie2 Fn2-3 domains, in which dots in the mouse Tie2 sequence indicate residues identical to human Tie2,
   c showing open-book views showing the interacting interfaces of Tie2 Fn2-3 (top) and chimeric hTAAB Fab (bottom) as surface representations, in which the color scheme of hTie2 Fn2-3 and chimeric hTAAB Fab is identical to that in FIG. 3a, but inverted for each binding surface, and six CDR loops of chimeric hTAAB Fab are represented by different color lines (bottom), and
   d showing a sequence comparison of the hTAAB heavy-chain (top) and light-chain (bottom) variable regions with the closest human and mouse variable region germline genes, in which dots in mouse and human germline sequences indicate residues identical to hTAAB, and in (b-d), hTie2 residues interacting with the heavy and light chains of chimeric hTAAB Fab are colored in dark orange and light orange, respectively, V730 interacting with both heavy and light chains is highlighted in red (b and c, top), and chimeric hTAAB Fab residues interacting with hTie2 Fn3 are colored in cyan (c, bottom, and d);

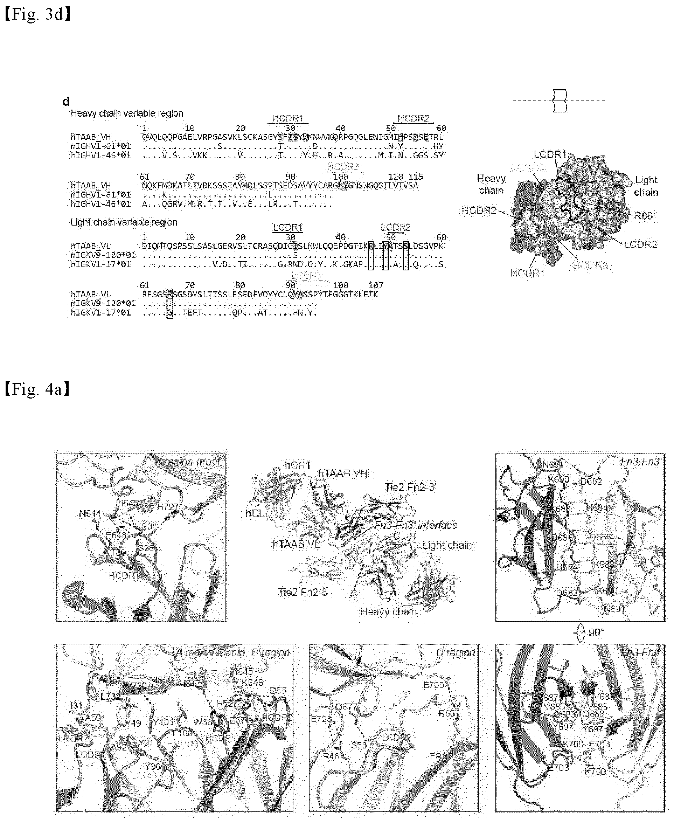

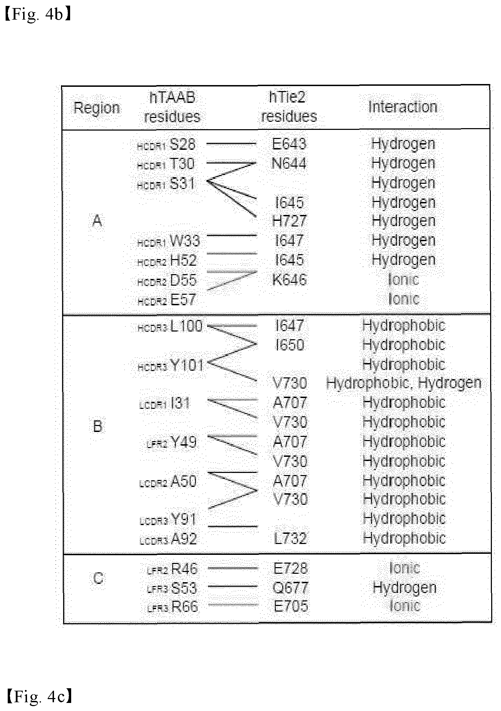

FIGs. 4a to 4d show results of analysis of the binding interface between chimeric hTAAB Fab and hTie2 Fn3,
   a showing a close-up view of key molecular interactions (A, B, and C regions), in which residues involved in hTie2 Fn3/chimeric hTAAB Fab interaction (red, green, and blue boxes) and hTie2 dimerization (black box) are represented as sticks and labeled, and hydrogen bonds and electrostatic interactions are represented as dashed lines,
   b showing key amino acid residues of A, B, and C regions involved in hTAAB binding, in which interaction residues of hTAAB are listed on the left and the corresponding interaction residues of Tie2 are listed on the right, and interactions between residue partners are represented in red (ionic interaction), black (hydrogen bond), and blue (hydrophobic interaction),
   c showing electrostatic potential of the hTie2 Fn2-3/chimeric hTAAB Fab complex calculated according to the Poisson-Boltzmann equation in PyMOL, in which the structures are indicated as open-book views and surface representations, with color maps reflecting the electrostatic properties (blue: positively charged, red: negatively charged), in which the interacting regions are highlighted by a yellow line, and
   d showing hydrophobic residues in hTie2 Fn2-3 and chimeric hTAAB Fab that appear on the surface, in which the interaction interface is represented by a black line;

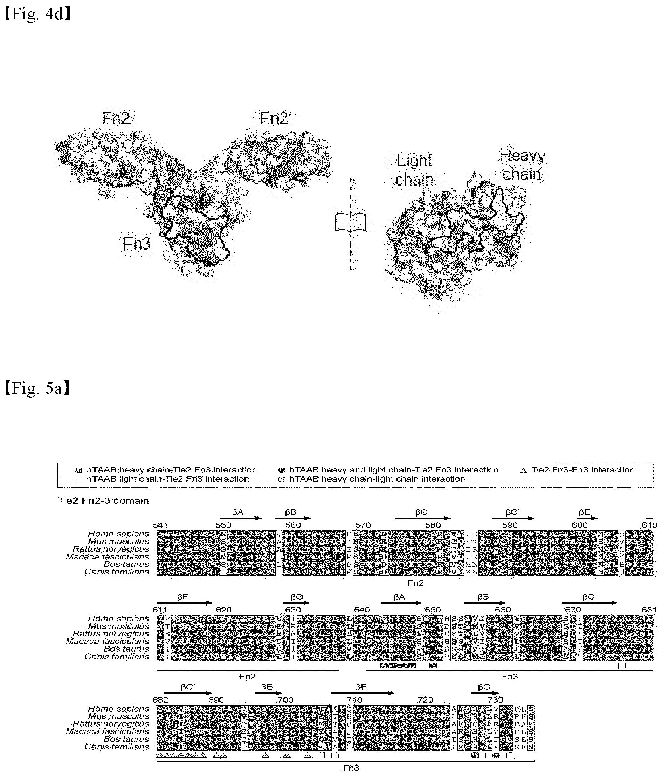

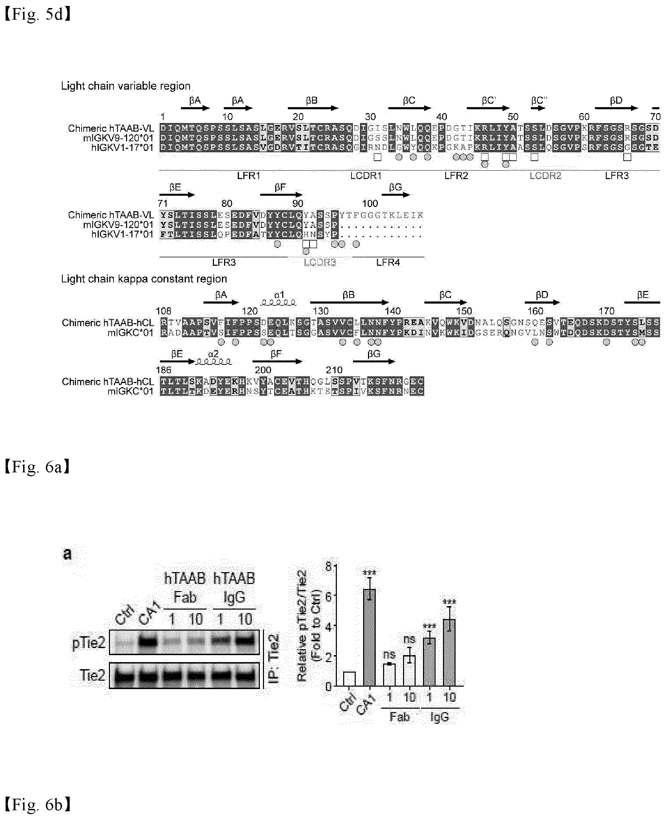

FIGs. 5a to 5d show results of sequence alignment of Tie2, Tie1 Fn2-Fn3 domain and chimeric hTAAB Fab,
   a showing sequence alignment of human (H. sapiens, UniProt: Q02763), mouse (M. musculus, UniProt: Q02858), rat (R. norvegicus, UniProt: D3ZCD0), monkey (M. fascicularis, UniProt: A0A2K5VRI3), bovine (B. taurus, UniProt: Q06807), and dog (C. familiaris, UniProt: F1P8U6) Tie2 Fn2-3,
   b showing sequence alignment of human (H. sapiens, UniProt: Q02763) and mouse (M. musculus, UniProt: Q02858) Tie2 Fn2-3, and human (H. sapiens, UniProt: P35590) and mouse (M. musculus, UniProt Q06806) Tie1 Fn2-3,
   c showing sequence alignment of heavy-chain variable region of chimeric hTAAB and closest mouse and human germline genes (top) and sequence alignment of heavy-chain gamma 1 constant region of chimeric hTAAB (hIGHV1*01) and closest mouse germline gene (bottom), and d showing sequence alignment of light-chain variable region of chimeric hTAAB and closest mouse and human germline genes (top) and sequence alignment of light-chain kappa constant region of chimeric hTAAB (hIGKC1*01) and closest mouse germline gene (bottom), and
   in (a-d), blue squares represent interacting residues in the chimeric hTAAB Fab heavy chain and hTie2 Fn3 interface, white squares represent interacting residues between chimeric hTAAB Fab light chain and hTie2 Fn3, the red circle represents residue V730 of hTie2, which interacts with both heavy and light chains, gray circles represent residues involved in interactions between the heavy and light chains of chimeric hTAAB Fab, the residues involved in dimeric interactions between Fn3 and Fn3 domains of Tie2 are represented as orange triangles, red boxes represent perfect sequence conservation, yellow boxes represent residues with more than 70% similarity based on physicochemical properties, secondary structural elements are noted above alignment with arrows (β-strands) and helix (α-helix), and the sequence alignment was created using T-Coffee (http://tcoffee.crg.cat) and ESPript servers (http://espript.ibcp.fr);

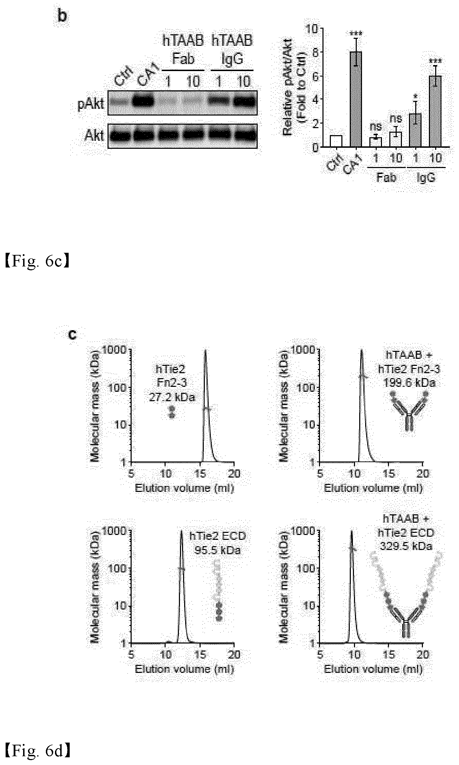

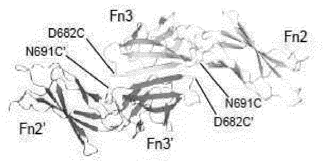

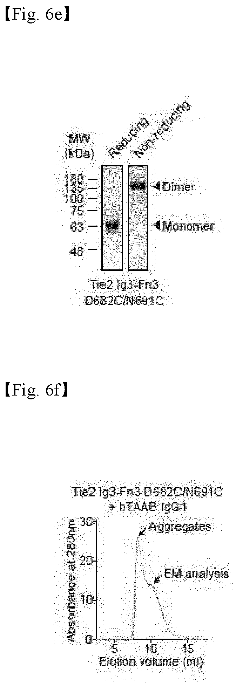

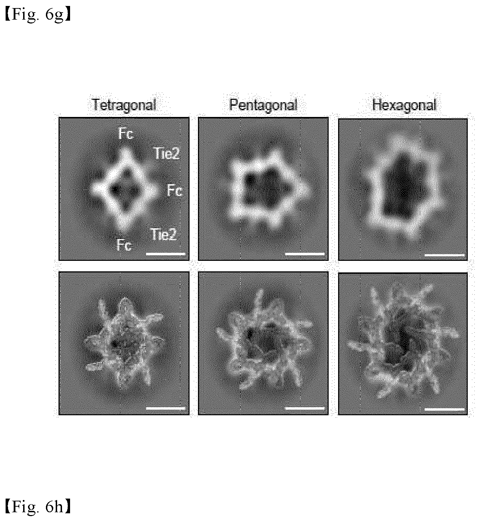

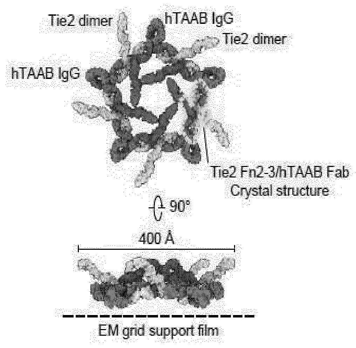

FIG. 6 shows results showing that hTAAB IgG binding mediates polygonal assembly of Tie2 dimers,
   a showing results of immunoblot analysis for relative phosphorylation ratios of Tie2 after treatment of HUVECs with chimeric hTAAB Fab or hTAAB mouse IgG1 (1 and 10 µg/ml) (left), with densitometric analysis of p-Tie2/Tie2 ratios (right; mean ± SD n = 3; ***p < 0.001 vs. control),
   b showing results of immunoblot analysis for relative phosphorylation ratios of Akt after treatment of HUVECs with chimeric hTAAB Fab or hTAAB IgG1 (1 and 10 µg/ml) (left), with densitometric analysis of p-Akt/Akt ratios (right; mean ± SD n = 3; *p < 0.05, ***p < 0.001 vs. control),
   c showing results of size-exclusion chromatography-multiangle light scattering (SEC-MALS) analysis of hTie2 Fn2-3, hTie2 ECD, hTAAB/hTie2 Fn2-3 complex, and hTAAB/hTie2 ECD complex, in which samples were run on a Superdex 200 Increase 10/300 GL column in 0.5 ml/min of PBS at protein concentrations of 3 mg/ml (hTie2 Fn2-3) or 1 mg/ml (all others), and the molecular weight (kDa) and absorbance at 280 nm are plotted against elution volume (ml),
   d showing design of the Tie2 dimeric mutant, in which residues D682 and N691 (yellow) on both ends of the antiparallel β-sheet interaction interface between Fn3 domains were mutated to cysteine to allow the formation of two disulfide bonds, producing a constitutive Tie2 Ig3-Fn3 dimer,
   e showing results of analysis of purified hTie2 Ig3-Fn3 D682C/N691C by SDS-PAGE and Coomassie blue staining under reducing and non-reducing conditions,
   f showing results of size-exclusion chromatography of hTie2 Ig3-Fn3 D682C/N691C in complex with hTAAB IgG1, in which purified hTie2 Ig3-Fn3 D682C/N691C was incubated with hTAAB IgG1 at a molar ratio of 2:1 (Tie2 monomer:hTAAB IgG1) for 2 hours and subjected to size-exclusion chromatography, and the fraction used for negative-stain EM and 2D class average is indicated as EM analysis,
   g showing representative 2D class averages of Tie2 dimeric mutant/hTAAB IgG1 complex (top), in which the cyclical higher-order structure of the Tie2 dimer/hTAAB IgG1 complex in 4-to-4, 5-to-5, and 6-to-6 assemblies was modeled based on the 2D class averages of respective tetragonal, pentagonal, and hexagonal closed-ring structures using crystal structures of the Tie2 Fn2-3/chimeric hTAAB Fab complex, Tie2 Ig1-Fn1 (PDB: 4K0V11), and Fc fragment of human IgG1 (PDB: 5VGP) (bottom), with scale bars of 20 nm, and
   h showing a 3D model of the polygonal 5-to-5 assembly of the Tie2 dimer/hTAAB IgG1 complex observed on a negative-stain EM grid, in which the 2:2 Tie2 Fn2-3/chimeric hTAAB Fab complex in an asymmetric unit of the crystal is represented by a red line;

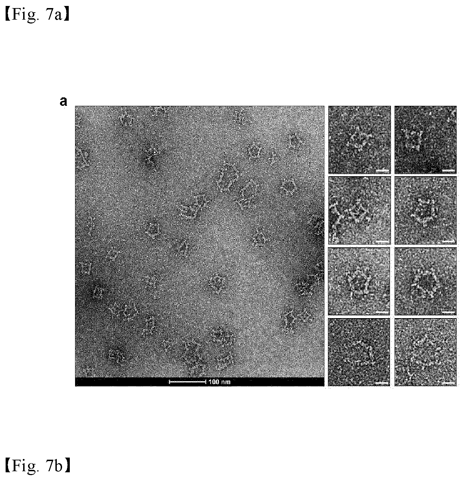

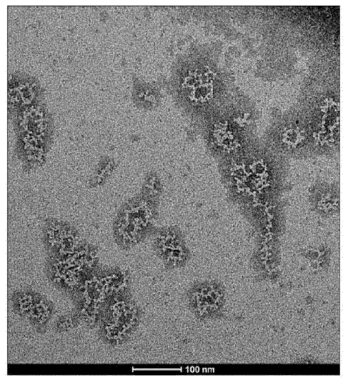

FIG. 7 shows results of negative-stain EM analysis of the Tie2 dimeric mutant/hTAAB IgG1 complex,
   a showing a representative micrograph of negative-stain EM analysis of purified hTie2 Ig3-Fn3 N691C/D682C in complex with hTAAB IgG1 (left; scale bars, 100 nm), with representative particle images of tetragonal, pentagonal, and hexagonal closed-ring structures (right; scale bars, 20 nm), and
   b showing a representative micrograph of negative-stain EM analysis of aggregation peak from size-exclusion chromatography of hTie2 Ig3-Fn3 N691C/D682C and hTAAB IgG1 complex, with scale bars of 100 nm;

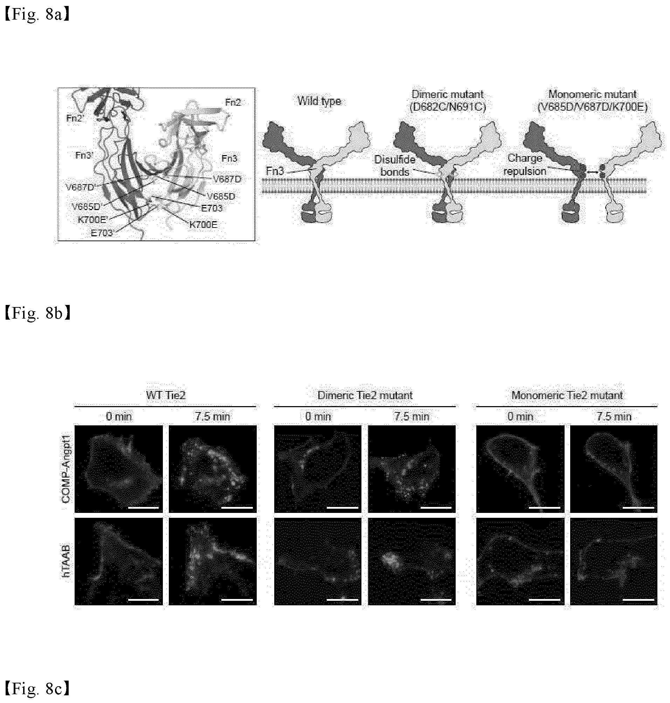

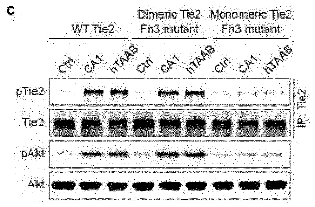

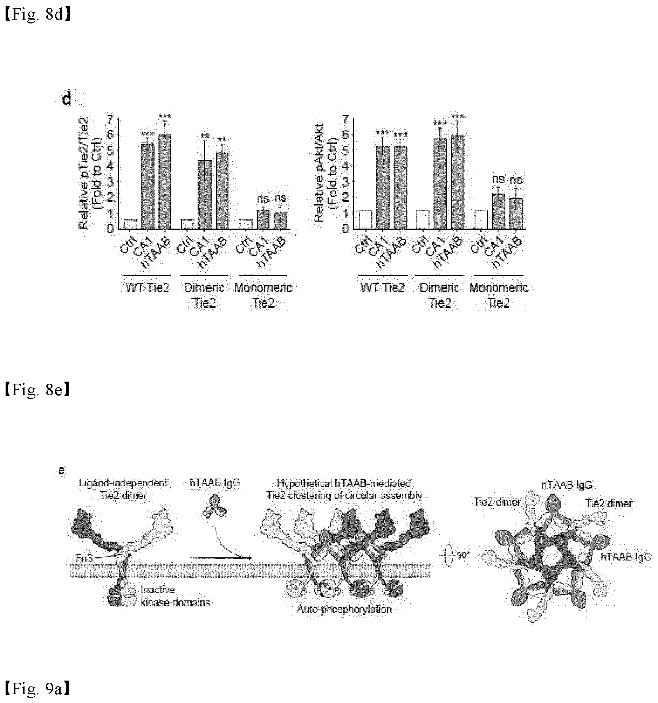

FIG. 8 shows results showing that ligand-independent Tie2 dimerization is essential for hTAAB-mediated Tie2 clustering and activation,
   a showing design of the Tie2 monomeric mutant, in which residues V685, V687, and K700 (yellow) at Fn3-Fn3' dimeric interface were mutated to negatively charged residues (V685D/V687D/K700E) for disrupting the Fn3-Fn3' dimeric interface through charge repulsion, producing a constitutive Tie2 monomer (left), with schematic representation of full-length Tie2-GFP WT, as well as constitutive Tie2 dimeric and monomeric mutants used for live-cell imaging and Tie2 activation assays in HEK293T cells (right),
   b showing confocal time-lapse images of Tie2 (full-length Tie2 WT, Tie2 dimeric mutant (D682C/N691C), or monomeric mutant (V685D/V687D/K700E)) in HEK293T cells after treatment with hTAAB and COMP-Ang1, in which images are represented at 60x magnification, with scale bars of 10 um,
   c and d showing results of immunoblot analysis for relative phosphorylation ratios of Tie2 and Akt in HEK293T cells transiently expressing WT Tie2, constitutive dimeric Tie2 mutant (D682C/N691C), or constitutive monomeric Tie2 mutant (V685D/V687D/K700E) after treatment with COMP-Angpt1 (1 ug/ml) or hTAAB IgG1 (10 ug/ml) for 1 hour (c), with densitometric analysis of p-Tie2/Tie2 and p-Akt/Akt ratios (d) (mean ± SD n = 3; *p < 0.05, **p < 0.01, ***p < 0.001 vs. control), and
   e showing a model for hTAAB-mediated Tie2 activation on the cell membrane, in which hTAAB IgG binds to the Fn3 domain of homodimeric Tie2 and induces clustering of ligand-independent Tie2 dimers into higher-order circular assemblies, and Tie2 clustering organizes inactive kinase dimers in a manner that is optimal for autophosphorylation between neighboring dimers;

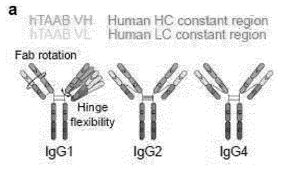

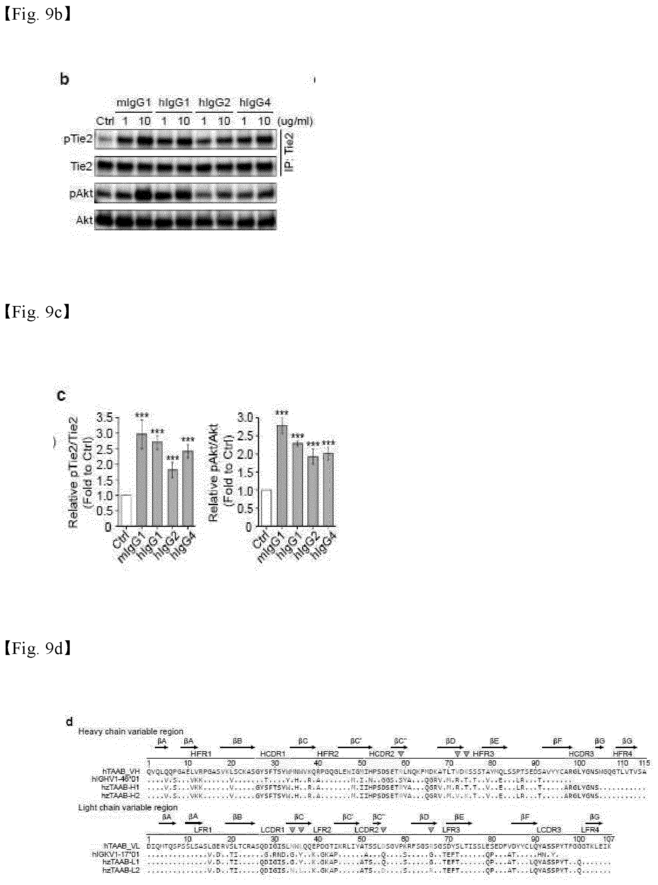

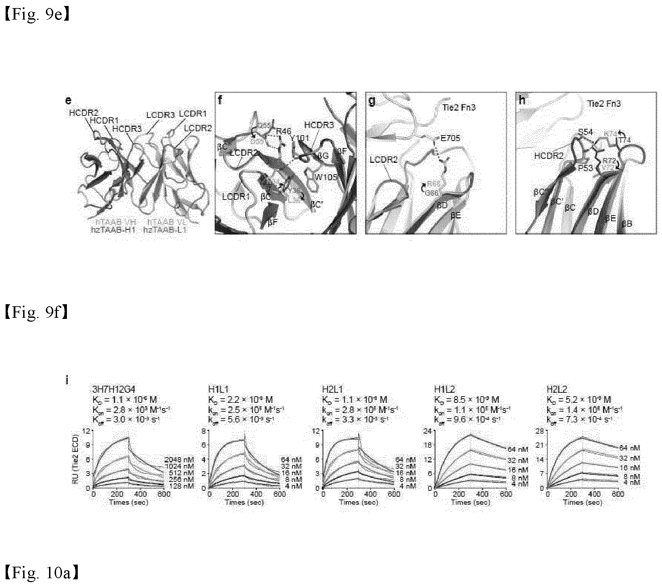

FIG. 9 shows structure-based humanization of hTAAB,
   a showing schematic representation of the chimeric hTAAB antibody in the form of IgG1, IgG2, and IgG4 subclasses, in which interchain disulfide bonds are represented by black lines, heavy- and light-chain variable regions are colored in dark orange and light orange, respectively, and heavy- and light-chain constant regions are colored in green and blue, respectively,
   b and c showing results of immunoblot analysis for relative phosphorylation ratios of Tie2 and Akt after treatment of HUVECs with hTAAB in the form of mouse IgG1 or human chimeric IgG1, IgG2, or IgG4 (1 and 10 µg/ml) (b), with densitometric analysis of p-Tie2/Tie2 and p-Akt/Akt ratios (c) (mean ± SD n = 3; *p < 0.05, **p < 0.01, ***p < 0.001 vs. control),
   d showing alignment of sequences of heavy- and light-chain variable regions of hTAAB and humanized TAAB (hzTAAB) with the closest human germline genes, in which dots in human germline and hzTAAB sequences indicate residues identical to parental hTAAB, CDRs of hTAAB and grafted CDRs of hzTAAB are colored in blue, back-mutated residues to parental hTAAB are colored in green and are represented as green triangles, and secondary structural elements (β-strands) are represented as arrows,
   e-h showing homology model structures of hzTAAB-H1L1 Fv superimposed on the chimeric hTAAB Fv structure adapted from the crystal structure of the Tie2 Fn2-Fn3/chimeric hTAAB Fab complex, in which the heavy and light chains of chimeric hTAAB are colored in dark orange and light orange, respectively, and heavy and light chains of hzTAAB-H1L1 are colored in purple and pink,
   f-h showing rationales for structure-based humanization of the Tie2-activating antibody, in which residues in the light chain critical for maintaining VH-VL pairing and CDR conformation (f) and affinity for Tie2 Fn3 (g) were back-mutated to the mouse hTAAB sequence, residues in the heavy chain critical for maintaining CDR conformation were back-mutated to the mouse hTAAB sequence (h), and mutations are represented as arrows and residues are colored the same as in (e), and
   i showing binding kinetics of humanized TAABs (hzTAAB-H1L1, hzTAAB-H2L1, hzTAAB-H1L2, and hzTAAB-H2L2, and the previously reported humanized construct 3H7H12G4) for hTie2 ECD measured by SPR analysis, in which the equilibrium dissociation constant (K_{D}, M) was calculated as the ratio of off-rate to on-rate (k_{off}/kₒₙ), kinetic parameters were determined with the global fitting function of Biacore Insight Evaluation Software using a 1:1-bidning model, and vertical dashed lines represent the start of the dissociation phase;

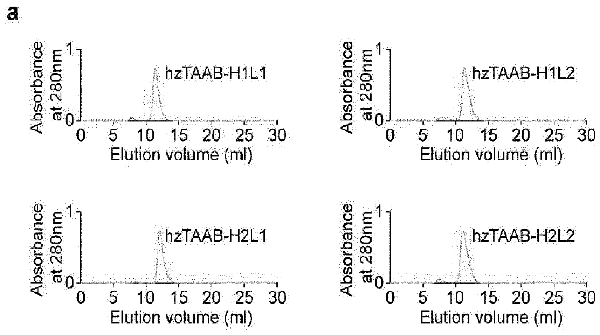

FIGs. 10a to 10c show results of analysis of humanized Tie2-activating antibodies,
   a showing results of size-exclusion chromatography of IgG1-based humanized Tie2-activating antibodies (hzTAAB H1L1, H1L2, H2L1, and H2L2),
   b showing results of analysis of concentrated elution fractions of hzTAABs by SDS-PAGE and Coomassie blue staining under reducing (left) and non-reducing (right) conditions, and
   c showing binding kinetics of hzTAABs for mTie2 Ig3-Fn3 measured by BLI (biolayer interferometry) analysis, with association (A) and dissociation (D) of mTie2 Ig3-Fn3 depicted as sensorgrams obtained using an Octet RED96 system, in which the indicated antibodies were immobilized on anti-human IgG Fc Capture (AHC) biosensors, followed by measurement of association by immersing the biosensors in wells containing a 1600 nM solution of mouse Tie2 Ig3-Fn3 and then measurement of dissociation by washing with kinetics buffer;

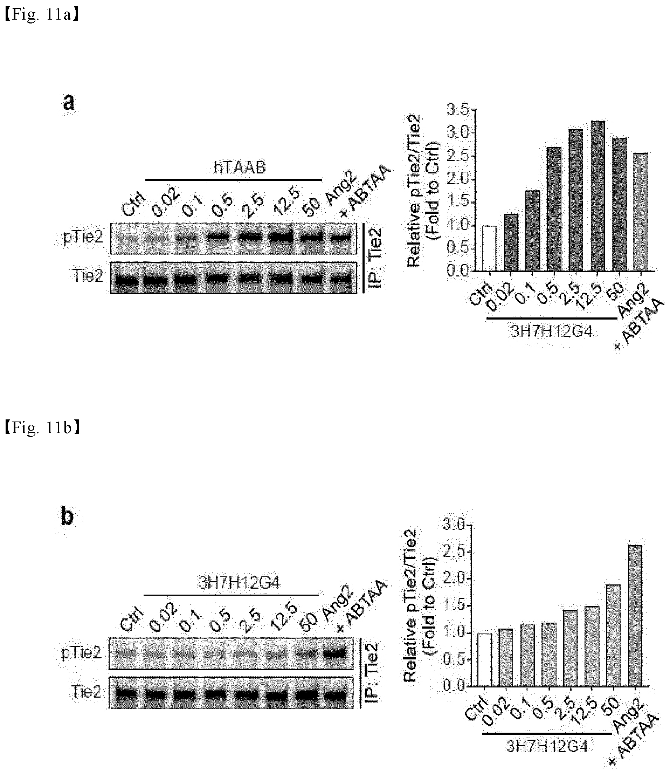

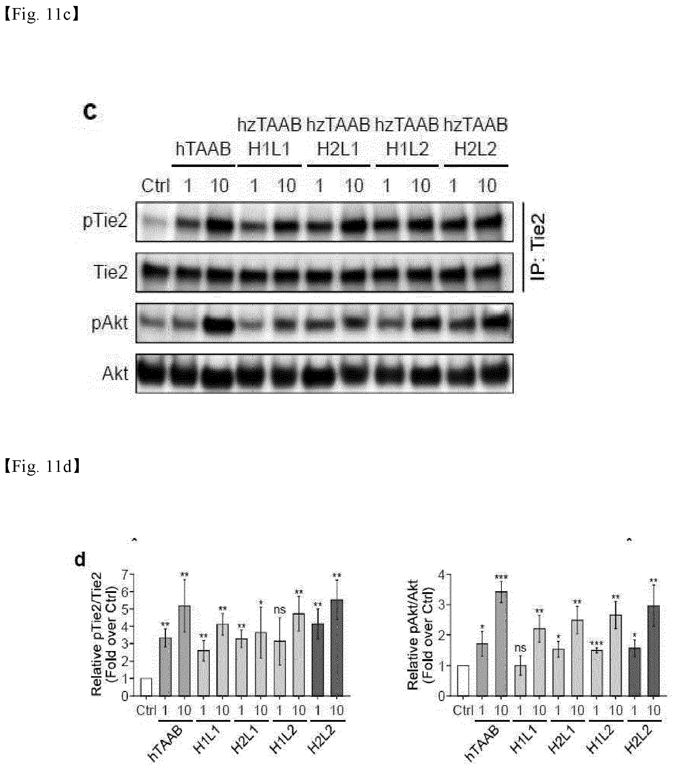

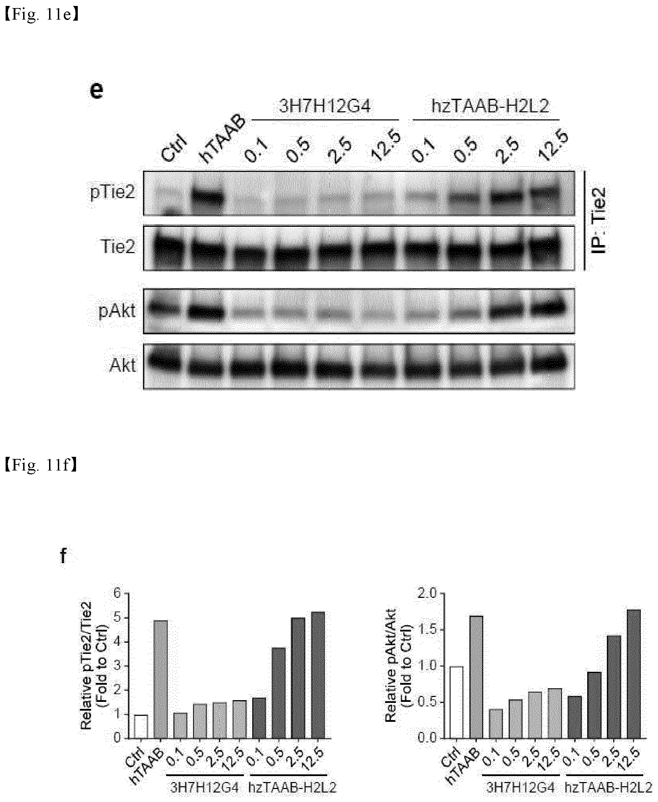

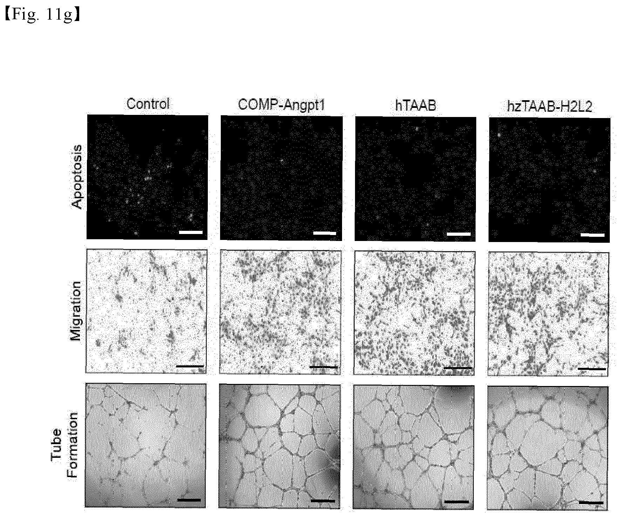

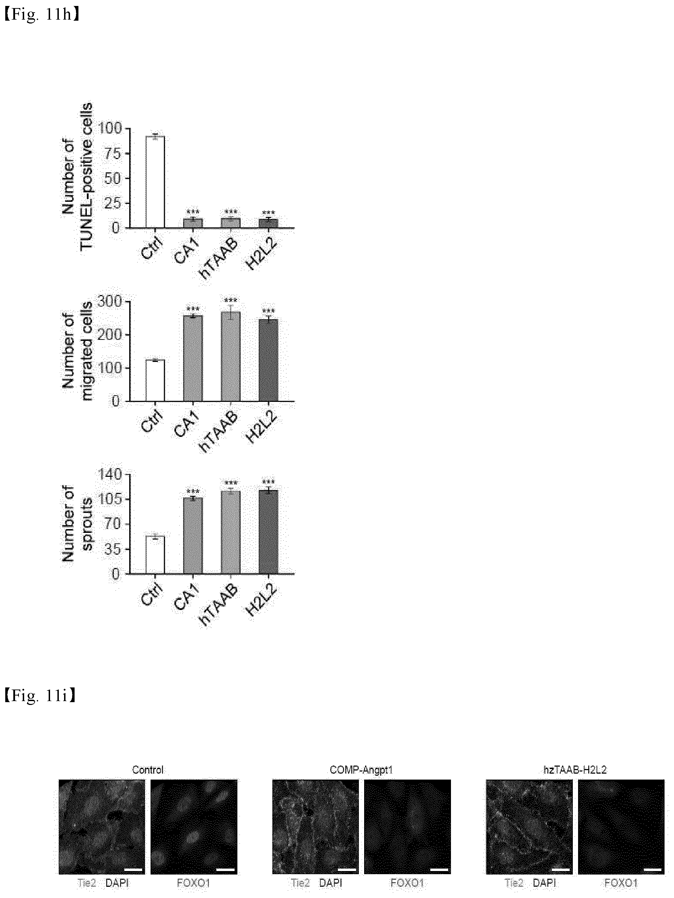

FIG. 11 shows results showing that humanized TAAB activates Tie2 and downstream signaling thereof in HUVECs,
   a and b showing results of immunoblot analysis for relative phosphorylation ratios of Tie2 after treatment of HUVECs with different concentrations (0.02, 0.1, 0.5, 2.5, 12.5, and 50 ug/ml) of hTAAB (a) or 3H7H12G4 (b), in which ABTAA (2.5 ug/ml) + Angpt2 was used as a positive control, with densitometric analysis of p-Tie2/Tie2 ratios (right),
   c and d showing results of immunoblot analysis for relative phosphorylation ratios of Tie2 and Akt after treatment of HUVECs with different concentrations (1 and 10 µg/ml) of humanized TAABs (hzTAAB-H1L1, H1L2, H2L1, and H2L2) or hTAAB (c), with densitometric analysis of p-Tie2/Tie2 and p-Akt/Akt ratios (d) (mean ± SD n = 3; *p < 0.05, **p < 0.01, ***p < 0.001 vs. control),
   e and f showing results of immunoblot analysis for relative phosphorylation ratios of Tie2 and Akt after treatment of HUVECs with 3H7H12G4 or hzTAAB-H2L2 at different concentrations (0.1, 0.5, 2.5, and 12.5 ug/ml), with densitometric analysis of p-Tie2/Tie2 and p-Akt/Akt ratios (f),
   g and h showing biological effects of hzTAAB-H2L2 in HUVECs, with effect of hzTAAB-H2L2 on serum deprivation-induced apoptosis of HUVECs (top), in which HUVECs were incubated in serum-free medium containing 10 µg/ml of hzTAA-H2L2, hTAAB, or 1 ug/ml of COMP-Angpt1, migration activity was measured using a modified Boyden chamber assay (middle), HUVECs were seeded in the upper layer of 8-um pore membranes, serum-free medium containing 10 µg/ml of hzTAA-H2L2, hTAAB, or 1 ug/ml of COMP-Angpt1 was added to the bottom chamber, migrated cells were fixed and stained after 9 hours of incubation, HUVECs were plated on Matrigel-coated wells and incubated in serum-free medium containing 10 µg/ml of hzTAA-H2L2, hTAAB, or 1 µg/ml of COMP-Angpt1, phase-contrast micrographs were obtained 7 hours after treatment, and tube-formation activity was measured as total tube length (bottom), with scale bars of 100 µm, and quantification (h) of TUNEL-positive cell, migrated HUVEC, and sprouts for (g) (mean ± SD n = 3; *p < 0.05, **p < 0.01, ***p < 0.001 vs. control), and
   i showing confocal images of HUVECs representing hzTAAB-H2L2-induced Tie2 translocation to cell-cell contacts and hzTAAB-H2L2-induced nuclear clearance of FOXO1, in which serum-starved HUVECs were treated with hTAAB-H2L2 (10 ug/ml) or COMP-Angpt1 (1 ug/ml) for 30 minutes, no treatment was not used as a control, and goat anti-human Tie2 and Alexa Fluor 488-conjugated donkey anti-goat antibodies were used for Tie2 visualization, and rabbit anti-FOXO1 and Alexa Fluor 594-conjugated donkey anti-rabbit antibodies were used for FOXO1 visualization, with scale bars of 20 um, DAPI: 4',6-diamidino-2-phenylindole; and

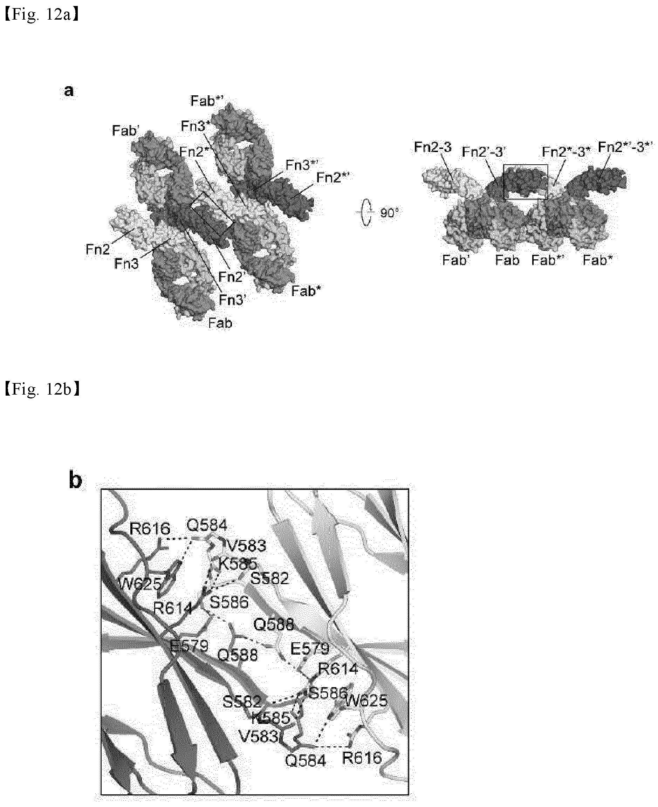

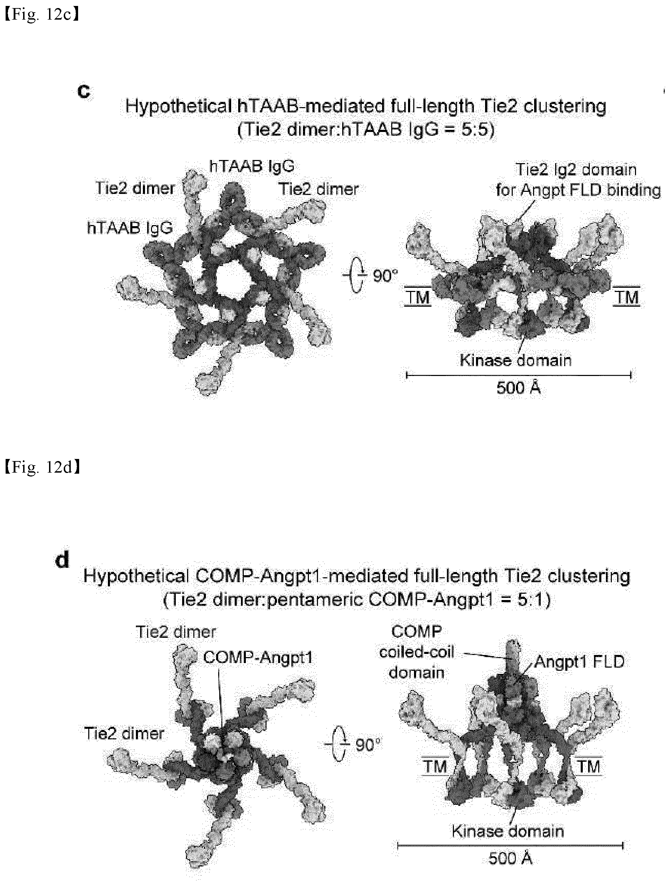

FIGs. 12a to 12d show models of full-length Tie2 clustering,
   a showing crystal packing interactions between chimeric hTAAB Fab/Tie2 Fn2-3 complexes, in which the color schemes for chimeric hTAAB Fab and Tie2 Fn2-3 are described in FIG. 3a, and lateral interactions between Fn2 domains introduced by crystal packing are represented as a black box,
   b showing a close-up view of crystal packing interactions shown in the black box in (A), in which the key binding residues mediated by crystal packing are indicated, and hydrogen bonds and electrostatic interactions are represented by dashed lines,
   c showing a proposed model for hTAAB-mediated Tie2 clustering, in which the cyclical higher-order structure of the full-length Tie2 dimer/hTAAB IgG1 complex in a 5-to-5 assembly was modeled based on a 2D class average of a pentagonal closed-ring structure using crystal structures of Tie2 Fn2-3/chimeric hTAAB Fab, Fc fragment of human IgG1 (PDB: 5VGP), and previously reported Tie2 constructs: Tie2 Ig1-Fn1 (PDB: 4K0V), Tie2 Fn1-3 (PDB: 5MYA), and Tie2 tyrosine kinase domain (PDB: 6MWE), and the transmembrane domain was adopted from the previously reported NMR structure of the dimerization motif of EGFR (PDB: 5LV6), and
   d showing a proposed model for COMP-Ang1-mediated Tie2 clustering, in which a cyclical complex of full-length Tie2 dimer with COMP-Ang1 complex in a 5-to-1 assembly was modeled based on the model of hTAAB-mediated full-length Tie2 clustering (C) using the model structure of Tie2s (C) and Ang1 FLD/Tie2 Ig1-Fn1 complex (PDB: 4K0V) and COMP coiled-coil domain (PDB: 1VDF) (C and D), and Tie2 Ig2 domains for Angpt FLD binding are colored in yellow.

### [Mode for Invention]

Unless otherwise defined, all technical and scientific terms used herein have the same meanings as typically understood by those skilled in the art to which the present invention belongs. In general, the nomenclature used herein is well known in the art and is typical.

The inventors of the present application developed a human Tie2-agonistic antibody hTAAB targeting the Tie2 Fn (membrane proximal fibronectin type III) domain. The Tie2/hTAAB complex structure operates in a new mode of Tie2 clustering.

hTAAB, which is a human Tie2-agonistic antibody, operates in a new mode of Tie2 clustering by forming a Tie2/hTAAB complex structure, and binds specifically to the Tie2 Fn3 domain, connecting Tie2 homodimers into a polygonal assembly. This structure is in contrast to the lateral Tie2 arrays observed in previous crystal lattices. Also, disruption of the Fn3-Fn3' dimeric interface inactivates the clustering signal of Tie2 induced by hTAAB. These results highlight the importance of Fn3-mediated Tie2 homodimerization for an hTAAB-induced Tie2 polygonal assembly and provide insight into how Tie2 agonists induce Tie2 clustering and activation. Also, the success of constructing humanized antibodies based on the structure of hTAAB creates potential clinical possibilities.

Accordingly, an aspect of the present invention pertains to a Tie2-agonistic antibody or antigen-binding fragment thereof, in which the antibody binds to the Ig3 Fn3 (membrane proximal fibronectin type III) domain of human Tie2, and by binding of the antibody, homodimeric Tie2 is formed into a polygonal assembly, and thus clustered and activated.

In addition, the present invention pertains to an antibody polygonal assembly including a Tie2-agonistic antibody or antigen-binding fragment thereof and formed with homodimeric Tie2 by binding of the antibody to the Ig3 Fn3 (membrane proximal fibronectin type III) domain of human Tie2.

As used herein, the term "antibody" refers to an antibody that specifically binds to Tie2. The scope of the present invention includes not only the complete antibody form that specifically binds to Tie2, but also antigen-binding fragments of the antibody molecule.

A complete antibody has a structure having two full-length light chains and two full-length heavy chains, and each light chain is linked to a heavy chain by a disulfide bond. The heavy-chain constant region has gamma (γ), mu (µ), alpha (α), delta (6), and epsilon (ε) types, and gamma 1 (γ1), gamma 2 (γ2), gamma 3 (γ3), gamma 4 (γ4), alpha 1 (α1), and alpha 2 (α2) subclasses. The constant region of the light chain has kappa (κ) and lambda (λ) types.

The antigen-binding fragment of an antibody or the antibody fragment is a fragment having an antigen-binding function, and includes Fab, F(ab'), F(ab')2, and Fv. Among antibody fragments, Fab has a structure having light-chain and heavy-chain variable regions, a light-chain constant region, and a first heavy-chain constant region (CH1), and has one antigen-binding site. Fab' differs from Fab in that Fab' has a hinge region including at least one cysteine residue at the C-terminus of the heavy-chain CH1 domain. The F(ab')2 antibody is produced by a disulfide bond between cysteine residues in the hinge region of Fab'. Fv is a minimal antibody fragment having only a heavy-chain variable region and a light-chain variable region. Two-chain Fv is configured such that a heavy-chain variable region and a light-chain variable region are linked by a non-covalent bond, and single-chain Fv (scFv) is configured such that a heavy-chain variable region and a light-chain variable region are generally linked by a covalent bond via a peptide linker therebetween, or are directly linked at the C-terminus, forming a dimeric structure, like the two-chain Fv. Such antibody fragments may be obtained using proteolytic enzymes (e.g., Fab may be obtained by restriction-cleaving a whole antibody with papain, and F(ab')2 may be obtained by restriction-cleaving a whole antibody with pepsin), or may be constructed through genetic recombination technology.

In an embodiment, the antibody according to the present invention is in Fv form (e.g., scFv) or in intact antibody form. Also, the heavy-chain constant region may be any one selected from among isotypes such as gamma (γ), mu (µ), alpha (α), delta (δ), and epsilon (ε). For example, the constant region may be gamma 1 (IgG1), gamma 3 (IgG3), or gamma 4 (IgG4). The light-chain constant region may be kappa or lambda type.

As used herein, the term "heavy chain" is understood to include a full-length heavy chain and fragments thereof, the full-length heavy chain including a variable region domain VH including an amino acid sequence having a variable region sequence sufficient to confer specificity to an antigen and three constant region domains CH1, CH2, and CH3. Also, the term "light chain" used herein is understood to include a full-length light chain and fragments thereof, the full-length light chain including a variable region domain VL including an amino acid sequence having a variable region sequence sufficient to confer specificity to an antigen and a constant region domain CL.

Examples of the antibody of the present invention include, but are not limited to, monoclonal antibodies, multispecific antibodies, human antibodies, humanized antibodies, chimeric antibodies, single-chain Fvs (scFVs), single-chain antibodies, Fab fragments, F(ab') fragments, disulfide-liked Fvs (sdFVs), anti-idiotype (anti-Id) antibodies, epitope-binding fragments of these antibodies, and the like.

A monoclonal antibody is an antibody obtained from a population of substantially homogeneous antibodies, in which the individual antibodies that make up the population are identical, except for possible naturally-occurring mutations that may be present at low frequency. A monoclonal antibody is highly specific and is induced against a single antigenic site. In contrast to typical (polyclonal) antibodies, which commonly include different antibodies directed against different determinants (epitopes), each monoclonal antibody is directed against a single determinant on the antigen.

The term "epitope" refers to a protein determinant to which an antibody is able to specifically bind. The epitope is usually composed of a group of chemically active surface molecules, for example amino acids or sugar side-chains, and generally has specific three-dimensional structural features and specific charge properties. Conformational and nonconformational epitopes are distinguished in that binding to the former, but not the latter, is lost in the presence of a denaturing solvent.

A non-human (e.g., murine) antibody in a "humanized" form is a chimeric antibody including at least one amino acid sequence (e.g., CDR sequence) derived from at least one non-human antibody (donor or source antibody) that contains a minimal sequence derived from a non-human immunoglobulin. In most cases, the humanized antibody is a human immunoglobulin (recipient antibody), in which a residue from the hypervariable region of a recipient is replaced with a residue from the hypervariable region of a non-human species (donor antibody) having the desired specificity, affinity, and capability, for example, mice, rats, rabbits, or non-human primates. For humanization, residues within at least one framework domain (FR) of the variable region of the recipient human antibody may be replaced with corresponding residues from a non-human species donor antibody. This helps maintain the proper three-dimensional configuration of the grafted CDR(s), thereby improving affinity and antibody stability. The humanized antibody may include a new residue that does not appear in an additional recipient antibody or donor antibody, for example, to further refine antibody performance.

A "human antibody" is a molecule derived from human immunoglobulin, and means that all of the amino acid sequences constituting the antibody including a complementarity-determining region and a framework region are composed of human immunoglobulin.

A portion of the heavy chain and/or light chain is identical to or homologous with the corresponding sequence in an antibody derived from a particular species or belonging to a particular antibody class or subclass, whereas the remaining chain(s) includes a "chimeric" antibody (immunoglobulin) that is identical to or homologous with the corresponding sequence in an antibody derived from another species or belonging to another antibody class or subclass, as well as fragments of such antibodies that exhibit the desired biological activity.

The "variable region" of the antibody as used herein is a light-chain or heavy-chain portion of an antibody molecule including the amino acid sequence of a complementarity-determining region (CDR; i.e., CDR1, CDR2, and CDR3) and a framework region (FR). VH refers to the variable region of a heavy chain, and VL refers to the variable region of a light chain.

The "complementarity-determining region" (CDR; i.e., CDR1, CDR2, and CDR3) is an amino acid residue of the antibody variable domain that is necessary for antigen binding. Each variable domain typically has three CDRs, identified as CDR1, CDR2, and CDR3.

The "framework region" (FR) is a variable domain residue other than the CDR residue. Each variable domain typically has four FRs, identified as FR1, FR2, FR3, and FR4.

The Tie2 antibody is monovalent or bivalent and contains a single chain or two chains. Functionally, binding affinity of the Tie2 antibody falls in the range of 10⁻⁵ M to 10⁻¹² M. For example, binding affinity of the Tie2 antibody may be 10⁻⁶ M to 10⁻¹² M, 10⁻⁷ M to 10⁻¹² M, 10⁻⁸ M to 10⁻¹² M, 10⁻⁹ M to 10⁻¹² M, 10⁻⁵ M to 10⁻¹¹ M, 10⁻⁶ M to 10⁻¹¹ M, 10⁻⁷ M to 10⁻¹¹ M, 10⁻⁸ M to 10⁻¹¹ M, 10⁻⁹ M to 10⁻¹¹ M, 10⁻¹⁰ M to 10⁻¹¹ M, 10⁻⁵ M to 10⁻¹⁰ M, 10⁻⁶ M to 10⁻¹⁰ M, 10⁻⁷ M to 10⁻¹⁰ M, 10⁻⁸ M to 10⁻¹⁰ M, 10⁻⁹ M to 10⁻¹⁰ M, 10⁻⁵ M to 10⁻⁹ M, 10⁻⁶ M to 10⁻⁹ M, 10⁻⁷ M to 10⁻⁹ M, 10⁻⁸ M to 10⁻⁹ M, 10⁻⁵ M to 10⁻⁸ M, 10⁻⁶ M to 10⁻⁸ M, 10⁻⁷ M to 10⁻⁸ M, 10⁻⁵ M to 10⁻⁷ M, 10⁻⁶ M to 10⁻⁷ M, or 10⁻⁵ M to 10⁻⁶ M.

According to a specific embodiment of the present application, the antibody may be Fab including a heavy-chain variable region (VH) including the sequence of SEQ ID NO: 1, a heavy-chain constant region (CH) including the sequence of SEQ ID NO: 3, and a light-chain variable region (VL) including the sequence of SEQ ID NO: 2, and a light-chain constant region (CL) including the sequence of SEQ ID NO: 4.

| | Heavy Chain Variable Region Sequence | No. |
|---|---|---|
| hTAAB Fab | QVQLQQPGAELVRPGASVKLSCKASGYSFTSYWMNWVKQRPGQGLEWIGMIHPS DSETRLNQKFMDKATLTVDKSSSTAYMQLSSPTSEDSAVYYCARGLYGNSWGQG TLVTVSA | 1 |
| | CAGGTCCAACTGCAGCAGCCTGGGGCTGAGCTGGTGAGGCCTGGAGCTTCAGT GAAGCTGTCCTGCAAGGCTTCTGGCTACTCCTTCACCAGCTACTGGATGAACTG GGTGAAGCAGAGGCCTGGACAAGGCCTTGAGTGGATTGGCATGATTCATCCTT CCGATAGTGAAACTAGGTTAAATCAGAAGTTCATGGACAAGGCCACATTGACTG TAGACAAATCCTCCAGCACAGCCTACATGCAGCTCAGCAGCCCGACATCTGAG GACTCTGCGGTCTATTACTGTGCTCGTGGCCTCTATGGTAACTCTTGGGGCCAA GGGACTCTGGTCACTGTCTCTGCA | 9 |
| | Light Chain Variable Region Sequence | |
| | DIQMTQSPSSLSASLGERVSLTCRASQDIGISLNWLQQEPDGTIKRLIYATSSLDSG VPKRFSGSRSGSDYSLTISSLESEDFVDYYCLQYASSPYTFGGGTKLEIK | 2 |
| | GACATCCAGATGACCCAGTCTCCATCCTCCTTATCTGCCTCTCTGGGAGAAAGA GTCAGTCTCACTTGTCGGGCAAGTCAGGACATTGGTATTAGCTTAAACTGGCTT CAGCAGGAACCAGATGGAACTATTAAACGCCTGATCTACGCCACATCCAGTTTA GATTCTGGTGTCCCCAAAAGGTTCAGTGGCAGTAGGTCTGGGTCAGATTATTCT CTCACCATCAGCAGCCTTGAGTCTGAAGATTTTGTAGACTATTACTGTCTACAAT ATGCTAGTTCTCCGTACACGTTCGGAGGGGGGACCAAGCTGGAAATAAAA | 10 |
| | human γ1 heavy chain constant region | |
| | AKTTPPSVYPLAPGSAAQTNSMVTLGCLVKGYFPEPVTVTWNSGSLSSGVHTFPAV LQSDLYTLSSSVTVPSSPRPSETVTCNVAHPASSTKVDKKIVPRDCG | 3 |
| | GCTAAAACCACACCTCCCAGCGTGTACCCTCTGGCCCCAGGATCAGCAGCCCA GACCAATAGCATGGTGACACTCGGATGCCTGGTAAAGGGCTATTTCCCTGAGCC TGTGACGGTCACTTGGAATAGTGGGAGTTTGTCTAGTGGTGTTCACACATTTCC CGCTGTCCTGCAGAGCGATTTATACACACTTAGCTCCTCTGTGACCGTGCCATCC TCTCCTAGACCGTCAGAAACTGTGACTTGTAACGTCGCCCATCCAGCATCATCC ACCAAAGTTGACAAGAAGATCGTTCCCAGAGACTGTGGC | 35 |
| | human κ light chain constant region | |
| | RADAAPTVSIFPPSSEQLTSGGASVVCFLNNFYPKDINVKWKIDGSERQNGVLNSW TDQDSKDSTYSMSSTLTLTKDEYERHNSYTCEATHKTSTSPIVKSFNRNEC | 4 |
| | CGCGCTGATGCCGCTCCTACCGTCTCCATATTTCCGCCCTCCTCAGAGCAGCTCA CATCAGGTGGGGCGTCCGTGGTGTGCTTTCTGAATAATTTCTATCCGAAGGATAT TAACGTCAAATGGAAAATTGACGGCTCGGAACGTCAAAACGGTGTTCTGAACT CCTGGACCGACCAGGATTCTAAGGATAGTACTTATAGTATGAGTAGCACATTGAC ACTTACTAAAGACGAATATGAGCGTCACAATAGCTACACCTGTGAGGCCACCCA TAAAACGAGCACCTCGCCGATCGTTAAGAGCTTTAATCGCAATGAATGC | 36 |

In a specific embodiment according to the present invention, a humanized antibody hzTAAB, such as hzTAAB-H1 or hzTAAB-L1, was constructed. The humanized antibody hzTAAB may include a heavy-chain variable region including the amino acid sequence of SEQ ID NO: 5 or SEQ ID NO: 7 and a light-chain variable region including the amino acid sequence of SEQ ID NO: 6 or SEQ ID NO: 8.

Specifically, in claim 3, the humanized antibody may include:
a heavy-chain variable region including the amino acid sequence of SEQ ID NO: 5 and a light-chain variable region including the amino acid sequence of SEQ ID NO: 6;
a heavy-chain variable region including the amino acid sequence of SEQ ID NO: 7 and a light-chain variable region including the amino acid sequence of SEQ ID NO: 6;
a heavy-chain variable region including the amino acid sequence of SEQ ID NO: 5 and a light-chain variable region including the amino acid sequence of SEQ ID NO: 8; or
a heavy-chain variable region including the amino acid sequence of SEQ ID NO: 7 and a light-chain variable region including the amino acid sequence of SEQ ID NO: 8.

| Antibody | Sequence (VH) | No. | Sequence (VL) | No. |
|---|---|---|---|---|
| hzTAAB-H1L1 | QVQLVQSGAEVKKPGASVKVSCKA SGYSFTSYWMHWVRQAPGQGLEW MGIIHPSDSETRYAQKFQGRVTMTR DTSTSTVYMELSSLRSEDTAVYYCA RGLYGNSWGQGTLVTVSS | 5 | DIQMTQSPSSLSASVGDRVTITC RASQDIGISLGWYQQKPGKAPK RLIYATSSLQSGVPSRFSGSGSG TEFTLTISSLQPEDFATYYCLQY ASSPYTFGQGTKLEIK | 6 |
| hzTAAB-H2L1 | QVQLVQSGAEVKKPGASVKVSCKA SGYSFTSYWMHWVRQAPGQGLEW MGIIHPSDSETRYAQKFQGRVTMTV DKSTSTVYMELSSLRSEDTAVYYCA RGLYGNSWGQGTLVTVSS | 7 | DIQMTQSPSSLSASVGDRVTITC RASQDIGISLGWYQQKPGKAPK RLIYATSSLQSGVPSRFSGSGSG TEFTLTISSLQPEDFATYYCLQY ASSPYTFGQGTKLEIK | 6 |
| hzTAAB-H1L2 | QVQLVQSGAEVKKPGASVKVSCKA SGYSFTSYWMHWVRQAPGQGLEW MGIIHPSDSETRYAQKFQGRVTMTR DTSTSTVYMELSSLRSEDTAVYYCA RGLYGNSWGQGTLVTVSS | 5 | DIQMTQSPSSLSASVGDRVTITC RASQDIGISLNWLQQKPGKAPK RLIYATSSLDSGVPSRFSGSRSG TEFTLTISSLQPEDFATYYCLQY ASSPYTFGQGTKLEIK | 8 |
| hzTAAB-H2L2 | QVQLVQSGAEVKKPGASVKVSCKA SGYSFTSYWMHWVRQAPGQGLEW MGIIHPSDSETRYAQKFQGRVTMTV DKSTSTVYMELSSLRSEDTAVYYCA RGLYGNSWGQGTLVTVSS | 7 | DIQMTQSPSSLSASVGDRVTITC RASQDIGISLNWLQQKPGKAPK RLIYATSSLDSGVPSRFSGSRSG TEFTLTISSLQPEDFATYYCLQY ASSPYTFGQGTKLEIK | 8 |

The antibody or antibody fragment according to the present invention may include not only the sequence of the anti-Tie2 antibody set forth herein, but also biological equivalents thereto, within a range that enables specific recognition of Tie2. For example, additional modifications may be made to the amino acid sequence of an antibody in order to further improve the binding affinity and/or other biological properties of the antibody. Such modifications include, for example, deletion, insertion, and/or substitution of the amino acid sequence residues of the antibody. The amino acid variations are based on the relative similarity of amino acid side-chain substituents with regard to, for example, hydrophobicity, hydrophilicity, charge, size, and the like. Based on analysis of the size, shape, and type of amino acid side-chain substituents, all of arginine, lysine, and histidine are positively charged residues, alanine, glycine, and serine have similar sizes, and phenylalanine, tryptophan, and tyrosine have similar shapes. Therefore, based on these considerations, arginine, lysine, and histidine may be regarded as biologically functional equivalents, alanine, glycine, and serine may be regarded as biologically functional equivalents, and phenylalanine, tryptophan, and tyrosine may be regarded as biologically functional equivalents.

Taking into consideration the above-described variations having equivalent biological activity, the amino acid sequence of the antibody of the present invention or the nucleic acid molecule encoding the same is to be understood as including a sequence showing substantial identity to the sequence set forth in the sequence number. When the sequence of the present invention and any other sequences are aligned so as to correspond to each other as closely as possible and the aligned sequence is analyzed using an algorithm commonly used in the art, "substantial identity" refers to sequences exhibiting at least 90% homology, most preferably at least 95% homology, at least 96% homology, at least 97% homology, at least 98% homology, or at least 99% homology. Alignment methods for sequence comparison are known in the art. The NCBI Basic Local Alignment Search Tool (BLAST) is accessible through NBCI and elsewhere, and may be used in conjunction with sequencing programs such as blastp, blasm, blastx, tblastn, and tblastx on the Internet. BLAST is available at www.ncbi.nlm.nih.gov/BLAST/. A method for comparing sequence homology using this program may be found at www.ncbi.nlm.nih.gov/BLAST/blast_help.html.

Based thereon, the antibody or antigen-binding fragment thereof according to the present invention may have 90%, 91%, 92%, 93%, 94%, 95%, 96%, 97%, 98%, 99%, or higher homology with a specified sequence or all of the sequences set forth herein. Such homology may be determined through sequence comparison and/or alignment using methods known in the art. For example, the percentage sequence homology of a nucleic acid or protein of the present invention may be determined using a sequence comparison algorithm (i.e., BLAST or BLAST 2.0), manual alignment, or visual inspection.

Another aspect of the present invention pertains to a nucleic acid encoding the antibody or antigen-binding fragment thereof.

The nucleic acid may include the sequence selected from the group consisting of SEQ ID NOs: 9 to 14.

An antibody or antigen-binding fragment thereof may be recombinantly produced by isolating a nucleic acid encoding the antibody or antigen-binding fragment thereof according to the present invention. The nucleic acid may be isolated and inserted into a replicable vector for further cloning (DNA amplification) or further expression. Based thereon, still another aspect of the present invention pertains to a vector including the nucleic acid.

Here, "nucleic acid" has a meaning comprehensively encompassing DNA (gDNA and cDNA) and RNA molecules, and nucleotides, which are the basic building blocks of nucleic acids, include not only natural nucleotides but also analogues in which sugar or base regions are modified. The sequences of the nucleic acids encoding the heavy- and light-chain variable regions of the present invention may be modified. Such modification includes addition, deletion, or non-conservative or conservative substitution of nucleotides.

DNA encoding the antibody is easily isolated or synthesized using typical techniques (e.g., using an oligonucleotide probe capable of specifically binding to DNA encoding the heavy and light chains of the antibody). Many vectors are available. A vector component generally includes, but is not limited to, at least one selected from among a signal sequence, an origin of replication, at least one marker gene, an enhancer element, a promoter, and a transcription termination sequence.

As used herein, the term "vector" refers to a means for expressing a target gene in a host cell, and includes a plasmid vector, a cosmid vector, a virus vector, such as a bacteriophage vector, an adenovirus vector, a retrovirus vector, and an adeno-associated virus vector, etc. In the vector, the nucleic acid encoding the antibody is operably linked with a promoter.

Here, "operably linked" means a functional linkage between a nucleic acid expression control sequence (e.g., a promoter, a signal sequence, or an array of transcription regulator binding sites) and a different nucleic acid sequence, whereby the control sequence serves to control the transcription and/or translation of the different nucleic acid sequence.

When a prokaryotic cell is used as a host, a strong promoter capable of promoting transcription (e.g., a tac promoter, lac promoter, lacUV5 promoter, lpp promoter, pLλ promoter, pRλ promoter, rac5 promoter, amp promoter, recA promoter, SP6 promoter, trp promoter, or T7 promoter), a ribosome-binding site for initiation of translation, and a transcription/translation termination sequence are generally included. In addition, for example, when a eukaryotic cell is used as a host, a promoter derived from the genome of a mammalian cell (e.g., a metallothionine promoter, β-actin promoter, human hemoglobin promoter, or human muscle creatine promoter) or a promoter derived from a mammalian virus (e.g., an adenovirus late promoter, vaccinia virus 7.5K promoter, SV40 promoter, cytomegalovirus (CMV) promoter, tk promoter of HSV, mouse mammary tumor virus (MMTV) promoter, LTR promoter of HIV, promoter of Moloney virus, promoter of Epstein-Barr virus (EBV), or promoter of Rous sarcoma virus (RSV)) may be used, and a polyadenylation sequence is generally used as a transcription termination sequence.

In some cases, the vector may be fused with another sequence in order to facilitate purification of the antibody expressed therefrom. Examples of the sequence that is fused therewith include glutathione S-transferase (Pharmacia, USA), maltose-binding protein (NEB, USA), FLAG (IBI, USA), and 6x His (hexa-histidine; Qiagen, USA).

The vector contains, as a selective marker, an antibiotic resistance gene that is commonly used in the art, for example, a gene conferring resistance to ampicillin, gentamicin, carbenicillin, chloramphenicol, streptomycin, kanamycin, geneticin, neomycin, or tetracycline.

Yet another aspect of the present invention pertains to cells transformed with the vector described above. Examples of the cells used to produce the antibody of the present invention may include, but are not limited to, prokaryotic cells, yeast cells, and higher eukaryotic cells.

Prokaryotic host cells, such as *Escherichia coli*, *Bacillus subtilis* and *Bacillus thuringiensis* as strains belonging to the genus *Bacillus*, *Streptomyces*, *Pseudomonas* (e.g., *Pseudomonas putida*), *Proteus mirabilis*, and *Staphylococcus* (e.g., *Staphylococcus carnosus*), may be used.

Here, animal cells are of greatest interest, and examples of useful host cell lines may include, but are not limited to, COS-7, BHK, CHO, CHOK1, DXB-11, DG-44, CHO/-DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S, and HT1080.

Still yet another aspect of the present invention pertains to a method of producing the antibody or antigen-binding fragment thereof including (a) culturing the cells described above and (b) recovering the antibody or antigen-binding fragment thereof from the cultured cells.

The cells may be cultured in various media. Any commercially available medium may be used as a culture medium without limitation. All other essential supplements known to those skilled in the art may be contained in appropriate concentrations. Culture conditions, such as temperature, pH, etc., are already in use with the host cells selected for expression, as will be apparent to those skilled in the art.

For recovery of the antibody or antigen-binding fragment thereof, impurities may be removed by, for example, centrifugation or ultrafiltration, and the resultant product may be purified using, for example, affinity chromatography, etc. Other additional purification techniques, such as anion or cation exchange chromatography, hydrophobic interaction chromatography, hydroxyapatite chromatography, and the like, may be used.

Even yet another aspect of the present invention pertains to a composition for preventing or treating an angiogenic disease including the antibody or antigen-binding fragment thereof as an active ingredient.

Here, "angiogenesis" refers to the formation or growth of new blood vessels from previously existing blood vessels, and "angiogenesis-related disease" refers to a disease related to the occurrence or progression of angiogenesis. A disease may fall within the scope of angiogenesis-related diseases without limitation so long as it may be treated with the antibody. Examples of the angiogenesis-related disease may include, but are not limited to, cancer, metastasis, diabetic retinopathy, retinopathy of prematurity, corneal graft rejection, macular degeneration, neovascular glaucoma, erythrosis, proliferative retinopathy, psoriasis, hemophilic arthritis, capillary formation of atherosclerotic plaques, keloid, wound granulation, vascular adhesion, rheumatoid arthritis, osteoarthritis, autoimmune diseases, Crohn's disease, restenosis, atherosclerosis, intestinal adhesions, cat scratch disease, ulcer, liver cirrhosis, nephritis, diabetic nephropathy, diabetes mellitus, inflammatory diseases, and neurodegenerative diseases. Moreover, the cancer may be selected from the group consisting of esophageal cancer, stomach cancer, large intestine cancer, rectal cancer, oral cancer, pharynx cancer, larynx cancer, lung cancer, colon cancer, breast cancer, uterine cervical cancer, endometrial cancer, ovarian cancer, prostate cancer, testicular cancer, bladder cancer, renal cancer, liver cancer, pancreatic cancer, bone cancer, connective tissue cancer, skin cancer, brain cancer, thyroid cancer, leukemia, Hodgkin's lymphoma, lymphoma, and multiple myeloid blood cancer, but is not limited thereto.

As used herein, the term "prevention or prophylaxis" refers to any action that inhibits or delays the onset of a disease of interest by administering the antibody or the composition according to the present invention. The term "treatment or therapy" refers to any action that ameliorates or alleviates symptoms of a disease of interest by administering the antibody or the composition according to the present invention.

The composition including the antibody of the present invention may be a pharmaceutical composition and may further include appropriate vehicles, excipients, or diluents typically used in the art.

The pharmaceutical composition including pharmaceutically acceptable vehicles may be provided in a variety of oral or parenteral dosage forms such as tablets, pills, powders, granules, capsules, suspensions, oral solutions, emulsions, syrups, sterile aqueous solutions, non-aqueous solutions, suspensions, lyophilizates, and suppositories. In regard thereto, the pharmaceutical composition of the present invention may be formulated in combination with diluents or excipients, such as fillers, thickeners, binders, wetting agents, disintegrants, surfactants, etc. Solid formulations for oral administration may be in the form of tablets, pills, powders, granules, capsules, etc. In connection with these solids, the compound of the present invention may be formulated in combination with at least one excipient, such as starch, calcium carbonate, sucrose, lactose, or gelatin. Lubricants such as simple excipients, magnesium stearate, talc, etc. may be additionally used. Liquid formulations for oral administration may be suspensions, oral solutions, emulsions, syrups, etc. Various excipients such as simple diluents such as water or liquid paraffin, wetting agents, sweeteners, aromatics, preservatives, etc. may be included in liquid formulations. Moreover, the pharmaceutical composition of the present invention may be in a parenteral dosage form such as a sterile aqueous solution, non-aqueous solvent, suspension, emulsion, lyophilizate, suppository, etc. Injectable propylene glycol, polyethylene glycol, vegetable oils such as olive oil, and esters such as ethyl oleate may be suitable for non-aqueous solvents and suspensions. The basic materials for suppositories include Witepsol, Macrogol, Tween 61, cacao butter, laurin butter, and glycerogelatin.

The composition of the present invention is administered in a pharmaceutically effective amount. As used herein, the term "pharmaceutically effective amount" refers to an amount of a pharmaceutical composition sufficient to treat a disease at a reasonable benefit/risk ratio applicable to any medical treatment. The effective amount is determined depending on various factors including severity of the disease to be treated, the patient's age and gender, type of disease, activity of the drug, sensitivity to the drug, administration time, administration route, secretion rate, treatment period, co-administration of the drug, and other parameters known in the art. The composition of the present invention may be administered alone or in combination with other therapeutics. As such, the composition may be administered sequentially or simultaneously with conventional therapeutics. Also, the composition may be administered in a single dose or multiple doses. Taking these factors into full consideration, it is important to administer the minimum amount sufficient to obtain a maximum effect without side effects, and this dose may be readily determined by an expert in the field. The dose of the pharmaceutical composition of the present invention is not particularly limited, but varies depending on various factors, including the patient's health status and body weight, severity of the disease, type of drug, administration route, and administration time. The composition may be administered in a single dose or multiple doses per day into mammals, including rats, mice, livestock, humans, etc., by typically accepted routes, for example, orally, intrarectally, intravenously, subcutaneously, intrauterinely, or intracerebrovascularly.

A further aspect of the present invention pertains to a method of inhibiting angiogenesis or a method of preventing or treating an angiogenesis-related disease, including administering the antibody or the composition to a subject in need thereof.

The method of the present invention includes administering a pharmaceutically effective amount of a pharmaceutical composition to a subject in need of inhibition of angiogenesis. The subject may be a mammal such as a dog, bovine, horse, rabbit, mouse, rat, chicken, or human, but is not limited thereto. The pharmaceutical composition may be administered parenterally, subcutaneously, intraperitoneally, intrapulmonarily, or intranasally, and, as necessary, by an appropriate method including intralesional administration for topical treatment. The preferred dose of the pharmaceutical composition of the present invention varies depending on various factors, including the subject's health status and body weight, severity of the disease, type of drug, administration route, and administration time, and may be readily determined by those skilled in the art.

Still a further aspect of the present invention pertains to a pharmaceutical composition for preventing or treating cancer including the antibody, or a method of preventing or treating cancer including administering the antibody or the composition to a subject in need thereof. Here, the terms "antibody", "prevention", and "treatment" are as described above.

The type of cancer is not limited, so long as it is able to be treated with the antibody of the present invention. Particularly, the antibody of the present invention is capable of preventing the onset or progression of cancer by inhibiting angiogenesis. Examples of cancer include, but are not limited to, esophageal cancer, stomach cancer, large intestine cancer, rectal cancer, oral cancer, pharynx cancer, larynx cancer, lung cancer, colon cancer, breast cancer, uterine cervical cancer, endometrial cancer, ovarian cancer, prostate cancer, testicular cancer, bladder cancer, renal cancer, liver cancer, pancreatic cancer, bone cancer, connective tissue cancer, skin cancer, brain cancer, thyroid cancer, leukemia, Hodgkin's lymphoma, lymphoma, and multiple myeloid blood cancer.

Also, the antibody of the present invention may be used in combination with other antibodies or biologically active agents or materials for various purposes. Yet a further aspect of the present invention pertains to a composition for co-administration with an additional therapeutic agent for an angiogenic disease, including the antibody or antigen-binding fragment thereof.

Examples of the additional therapeutic agent for an angiogenic disease may include anti-angiogenic drugs, anti-inflammatory drugs, and/or anticancer drugs. Thereby, these may overcome each other's resistance and improve efficacy.

When the composition according to the present invention is co-administered with an additional therapeutic agent for an angiogenic disease, the Tie2 antibody and the additional therapeutic agent for an angiogenic disease may be administered sequentially or simultaneously. For example, an anti-angiogenic drug, an anti-inflammatory drug, and/or an anticancer drug may be administered to a subject and then the composition including the Tie2 antibody or antigen-binding fragment thereof as an active ingredient may be administered thereto, or the composition may be administered to a subject and then an anti-angiogenic drug, an anti-inflammatory drug, and/or an anticancer drug may be administered thereto. In some cases, the composition may be administered to a subject simultaneously with an anti-angiogenic drug, an anti-inflammatory drug, and/or an anticancer drug.

### Examples

A better understanding of the present invention may be obtained through the following examples. These examples are merely set forth to illustrate the present invention, and are not to be construed as limiting the scope of the present invention, as will be apparent to those of ordinary skill in the art.

### Example 1. Production of Tie2-activating mouse monoclonal antibody

### 1.1 Expression and purification of recombinant protein

Recombinant Tie2 protein for mouse immunization was produced by cloning the gene encoding hTie2 Ig3-Fn3 (human Tie2 residues 349-738, GenBank accession number: AAH35514.2) into the pFuse-hIgG1-Fc vector (pFuse-hg1fc1, InvivoGen), followed by transient expression in Expi293F cells.

### Human Tie2 Ig3-Fn3 (349T-738P)

**Amino acid sequence (SEQ ID NO: 19)** 
TPKIVDLPDHIEVNSGKFNPICKASGWPLPTNEEMTLVKPDGTVLHPKDFN
HTDHFSVAIFTIHRILPPDSGVWVCSVNTVAGMVEKPFNISVKVLPKPLNAPNVID
TGHNFAVINISSEPYFGDGPIKSKKLLYKPVNHYEAWQHIQVTNEIVTLNYLEPRT
EYELCVQLVRRGEGGEGHPGPVRRFTTASIGLPPPRGLNLLPKSQTTLNLTWQPIF
PSSEDDFYVEVERRSVQKSDQQNIKVPGNLTSVLLNNLHPREQYVVRARVNTKAQG
EWSEDLTAWTLSDILPPQPENIKISNITHSSAVISWTILDGYSISSITIRYKVQGK
NEDQHVDVKIKNATITQYQLKGLEPETAYQVDIFAENNIGSSNPAFSHELVTLPES
QAP
**DNA sequence (SEQ ID NO: 20)**

ACCCCAAAGATAGTGGATTTGCCAGATCATATAGAAGTAAACAGTGGTAAA

TTTAATCCCATTTGCAAAGCTTCTGGCTGGCCGCTACCTACTAATGAAGAAATGAC
CCTGGTGAAGCCGGATGGGACAGTGCTCCATCCAAAAGACTTTAACCATACGGATC
ATTTCTCAGTAGCCATATTCACCATCCACCGGATCCTCCCCCCTGACTCAGGAGTT
TGGGTCTGCAGTGTGAACACAGTGGCTGGGATGGTGGAAAAGCCCTTCAACATTTC
TGTTAAAGTTCTTCCAAAGCCCCTGAATGCCCCAAACGTGATTGACACTGGACATA
ACTTTGCTGTCATCAACATCAGCTCTGAGCCTTACTTTGGGGATGGACCAATCAAA
TCCAAGAAGCTTCTATACAAACCCGTTAATCACTATGAGGCTTGGCAACATATTCA
AGTGACAAATGAGATTGTTACACTCAACTATTTGGAACCTCGGACAGAATATGAAC
TCTGTGTGCAACTGGTCCGTCGTGGAGAGGGTGGGGAAGGGCATCCTGGACCTGTG
AGACGCTTCACAACAGCTTCTATCGGACTCCCTCCTCCAAGAGGTCTAAATCTCCT
GCCTAAAAGTCAGACCACTCTAAATTTGACCTGGCAACCAATATTTCCAAGCTCGG
AAGATGACTTTTATGTTGAAGTGGAGAGAAGGTCTGTGCAAAAAAGTGATCAGCAG
AATATTAAAGTTCCAGGCAACTTGACTTCGGTGCTACTTAACAACTTACATCCCAG
GGAGCAGTACGTGGTCCGAGCTAGAGTCAACACCAAGGCCCAGGGGGAATGGAGTG
AAGATCTCACTGCTTGGACCCTTAGTGACATTCTTCCTCCTCAACCAGAAAACATC
AAGATTTCCAACATTACACACTCCTCGGCTGTGATTTCTTGGACAATATTGGATGG
CTATTCTATTTCTTCTATTACTATCCGTTACAAGGTTCAAGGCAAGAATGAAGACC
AGCACGTTGATGTGAAGATAAAGAATGCCACCATCACTCAGTATCAGCTCAAGGGC
CTAGAGCCTGAAACAGCATACCAGGTGGACATTTTTGCAGAGAACAACATAGGGTC
AAGCAACCCAGCCTTTTCTCATGAACTGGTGACCCTCCCAGAATCTCAAGCACCA

Particularly, Expi293F cells (2×10⁶ cells/ml) were cultured in Expi293 expression medium (A1435103, ThermoFisher) and then transfected with a plasmid encoding hTie2 Ig3-Fn3 using an ExpiFectamine293 transfection kit (A14524, ThermoFisher). The cells were cultured for 5 days at 37°C and 8% CO₂ in a shaking incubator (orbital shaker, 125 rpm). After removal of cells by centrifugation, the culture supernatant including the secreted hTie2 Ig3-Fn3-Fc fusion protein was purified on an AKTA purification system (GE Healthcare) equipped with a HiTrap MabSelect SuRe affinity column (11003494, GE Healthcare). The purified hTie2Ig3-Fn3-Fc fusion protein was concentrated using an Amicon Ultra centrifugal filter (UFC8030, Millipore), and the buffer was replaced with PBS. The Fc tag of the fusion protein was removed by thrombin cleavage (27-0846-01, GE Healthcare) at 22°C for 18 hours. The hTie2 Ig3-Fn3 protein was further purified by removing the cleaved Fc tag on a HiTrap MabSelect SuRe affinity column.

To prepare human Tie2 for crystallization, the gene encoding hTie2 Fn2-3 (residues 541-735) (described below) was cloned into pET-28a (69864, Novagen) and expressed in *E. coli* BL21 (DE3) RIL (230240, Agilent Technologies).

### Human Tie2 Fn2-3 (541I-735S)

**Amino acid sequence (SEQ ID NO: 21)** 
IGLPPPRGLNLLPKSQTTLNLTWQPIFPSSEDDFYVEVERRSVQKSDQQNI

KVPGNLTSVLLNNLHPREQYVVRARVNTKAQGEWSEDLTAWTLSDILPPQPENIKI
 
SNITHSSAVISWTILDGYSISSITIRYKVQGKNEDQHVDVKIKNATITQYQLKGLE

PETAYQVDIFAENNIGSSNPAFSHELVTLPES
**DNA sequence (SEQ ID NO: 22)**

ATCGGACTCCCTCCTCCAAGAGGTCTAAATCTCCTGCCTAAAAGTCAGACC
ACTCTAAATTTGACCTGGCAACCAATATTTCCAAGCTCGGAAGATGACTTTTATGT
TGAAGTGGAGAGAAGGTCTGTGCAAAAAAGTGATCAGCAGAATATTAAAGTTCCAG
GCAACTTGACTTCGGTGCTACTTAACAACTTACATCCCAGGGAGCAGTACGTGGTC
CGAGCTAGAGTCAACACCAAGGCCCAGGGGGAATGGAGTGAAGATCTCACTGCTTG
GACCCTTAGTGACATTCTTCCTCCTCAACCAGAAAACATCAAGATTTCCAACATTA
CACACTCCTCGGCTGTGATTTCTTGGACAATATTGGATGGCTATTCTATTTCTTCT
ATTACTATCCGTTACAAGGTTCAAGGCAAGAATGAAGACCAGCACGTTGATGTGAA
GATAAAGAATGCCACCATCACTCAGTATCAGCTCAAGGGCCTAGAGCCTGAAACAG
CATACCAGGTGGACATTTTTGCAGAGAACAACATAGGGTCAAGCAACCCAGCCTTT
TCTCATGAACTGGTGACCCTCCCAGAATCT

The cells were allowed to grow at 37°C in LB medium supplemented with 50 ug/ml kanamycin until OD600 was 0.4. Protein expression was induced in 0.05 mM IPTG (isopropyl β-d-1-thiogalactopyranoside) and cultured at 18°C for 15 hours. The cells were recovered by centrifugation, resuspended in lysis buffer (20 mM HEPES, pH 7.5, and 200 mM NaCl), and lysed by sonication on ice. After removal of cell debris by centrifugation (13,000x g at 4°C for 0.5 hours), the supernatant was applied to a Ni-NTA agarose affinity column (30210, QIAGEN). After washing with wash buffer (20 mM HEPES, pH 7.5, 200 mM NaCl, and 50 mM imidazole) equivalent to 5 times the column volume, proteins were eluted with elution buffer (20 mM HEPES, pH 7.5, 200 mM NaCl, and 400 mM imidazole), and further purified by size-exclusion chromatography using a HiLoad16/600 Superdex 200 pg column (28-9893-35, GE Healthcare) equilibrated with 20 mM HEPES, pH 7.5, and 200 mM NaCl. To prepare chimeric hTAAB Fab for crystallization, the synthesized heavy and light chains for chimeric hTAAB Fab were cloned into a modified pBAD expression vector for periplasmic secretion. *E. coli* Top10F (Invitrogen) cells were transformed with plasmid pBAD-Fab and then allowed to grow at 37°C in LB medium supplemented with 100 ug/ml ampicillin. Protein expression was induced with 0.2% arabinose at OD600 of 1.0, and the cells were allowed to grow at 30°C for 15 hours. The cells were recovered by centrifugation, resuspended in lysis buffer (20 mM HEPES, pH 7.5, and 200 mM NaCl), and lysed by sonication on ice. After removal of cell debris by centrifugation (13,000x g at 4°C for 30 minutes), the supernatant including soluble chimeric hTAAB Fab was applied to a Ni-NTA agarose affinity chromatography column (QIAGEN) and washed with wash buffer (20 mM HEPES, pH 7.5, 200 mM NaCl, and 50 mM imidazole) equivalent to 5 times the column volume. Proteins were eluted with elution buffer (20 mM HEPES, pH 7.5, 200 mM NaCl, and 400 mM imidazole) and further purified by size-exclusion chromatography using a HiLoad16/600 Superdex 200 pg column equilibrated with 20 mM HEPES, pH 7.5, and 200 mM NaCl. Purified proteins and antibodies were dispensed and stored at -80°C.

### 1.2 Immunization and production of B-cell hybridoma

Five-week-old BALB/c mice were immunized with purified hTie2-Ig3-Fn3 (100 ug/injection) mixed with adjuvant twice a week for 6 weeks. Anti-Tie2 antibodies in the sera of immunized mice were assessed by hTie2 ELISA (enzyme-linked immunosorbent assay). When antibody titers (at a 1:5000 dilution) adequately increased (OD > 1.0), B lymphocytes were isolated from the spleen of immunized mice and fused with cultured myeloma cells (SP2/0). The fused cells were cultured in HAT (hypoxanthine, aminopterin, and thymidine) medium, and hybridoma cells were selected and cultured with only fused myeloma cells and B lymphocytes.

B-cell hybridoma was maintained in DMEM (Dulbecco's Modified Eagle Medium) supplemented with 10% fetal bovine serum (FBS), penicillin (100 U/ml), and streptomycin (100 mg/ml). In order to produce anti-Tie2 antibodies in B-cell hybridoma, cells were washed with PBS and cultured in serum-free medium (SFM, 12045-076, Gibco) for 3 days.

The surviving hybridoma cells were dispensed in 96-well plates, and the culture supernatant was tested by hTie2 ELISA. For clone selection by limiting dilution, hybridoma pools showing + signals were selected.

### 1.3 Sequencing of DNA encoding monoclonal antibody

Hybridoma cells (2×10⁶ cells/ml) were cultured in DMEM containing 10% FBS, and total RNA was acquired using the RNeasy mini kit (Qiagen). RNA concentration was measured and cDNA was synthesized by reverse transcription (RT). Heavy- and light-chain variable region gene sequences were subjected to PCR using a Mouse Ig-Primer set (Novagen) and the synthesized cDNA as a template at 94°C for 5 minutes, followed by 35 cycles of 94°C for 1 minute, 50°C for 1 minute, 72°C for 2 minutes, and then 72°C for 6 minutes with gradual cooling to 4°C. The PCR product obtained from each reaction was cloned into a TA vector and subjected to DNA sequencing, yielding nucleotide sequences encoding the heavy- and light-chain variable regions of each antibody.

### 1.4 Production of Tie2-activating mouse monoclonal antibody

The ECD of Tie2 forms dimers through membrane-proximal Fn3, regardless of ligand binding. Multimeric Angpt1 binding to ligand-binding domains (LBDs) crosslinks these preformed Tie2 dimers into higher-order oligomers or "Tie2 clusters" for Tie2 activation and downstream signaling. However, the LBDs of preformed Tie2 homodimers are too far away (~260 Å) for a single antibody targeting LBD to induce Tie2 clustering. Based on this concept, an anti-Tie2 antibody binding to the membrane-proximal Tie2 Fn domain was postulated to induce Tie2 oligomerization and activation, like multimeric Angpt1.

hTAAB, which is an hTie2-activating mouse monoclonal antibody that targets Ig3-Fn3 of hTie2, not mTie2, was produced.

| Antibody | Variable Region Sequence | No . |
|---|---|---|
| hTAAB | Heavy Chain Variable Region Sequence | |
| | QVQLQQPGAELVRPGASVKLSCKASGYSFTSYWMNWVKQRPGQGLEWIGMI HPSDSETRLNQKFMDKATLTVDKSSSTAYMQLSSPTSEDSAVYYCARGLYGNS WGQGTLVTVSA | 1 |
| | CAGGTCCAACTGCAGCAGCCTGGGGCTGAGCTGGTGAGGCCTGGAGCTTCA GTGAAGCTGTCCTGCAAGGCTTCTGGCTACTCCTTCACCAGCTACTGGATGA ACTGGGTGAAGCAGAGGCCTGGACAAGGCCTTGAGTGGATTGGCATGATTC ATCCTTCCGATAGTGAAACTAGGTTAAATCAGAAGTTCATGGACAAGGCCAC ATTGACTGTAGACAAATCCTCCAGCACAGCCTACATGCAGCTCAGCAGCCCG ACATCTGAGGACTCTGCGGTCTATTACTGTGCTCGTGGCCTCTATGGTAACTC TTGGGGCCAAGGGACTCTGGTCACTGTCTCTGCA | 9 |
| | Light Chain Variable Region Sequence | |
| | DIQMTQSPSSLSASLGERVSLTCRASQDIGISLNWLQQEPDGTIKRLIYATSSLDS GVPKRFSGSRSGSDYSLTISSLESEDFVDYYCLQYASSPYTFGGGTKLEIK | 2 |
| | GACATCCAGATGACCCAGTCTCCATCCTCCTTATCTGCCTCTCTGGGAGAAA GAGTCAGTCTCACTTGTCGGGCAAGTCAGGACATTGGTATTAGCTTAAACTG GCTTCAGCAGGAACCAGATGGAACTATTAAACGCCTGATCTACGCCACATCC AGTTTAGATTCTGGTGTCCCCAAAAGGTTCAGTGGCAGTAGGTCTGGGTCA GATTATTCTCTCACCATCAGCAGCCTTGAGTCTGAAGATTTTGTAGACTATTA CTGTCTACAATATGCTAGTTCTCCGTACACGTTCGGAGGGGGGACCAAGCTG GAAATAAAA | 10 |

### Example 2. Confirmation of two hTAAB Fabs bound to V-shaped Tie2 dimer by crystal structure

Human-mouse chimeric IgG1, IgG2, and IgG4 antibodies of Tie2-activating antibody hTAAB were produced by cloning the heavy- or light-chain variable regions (VH or VL) of mouse hTAAB into backbone vectors expressing the human heavy- or light-chain constant regions (CH or CL). A DNA fragment encoding the heavy-chain variable region of mouse hTAAB was synthesized as the sequence "EcoRV-signal sequence-VH-NheI" (Bioneer, Inc). The synthesized DNA fragment was digested with EcoRV and NheI and then cloned into pFUSE-CHIg-hG1 (IgG1 isotype) and pFUSE-CHIg-hG2 (IgG2 isotype) vectors (InvivoGen). In order to construct a human IgG4 chimeric antibody, DNA encoding the heavy-chain variable region (VH) of mouse hTAAB was amplified by PCR using primers including EcoRI and NheI sites for cloning. Then, the PCR product was subcloned into a modified pOptiVEC-TOPO vector expressing the heavy-chain constant region (CH) of human IgG4 antibody. The chimeric light-chain expression vector was constructed by PCR-amplifying a DNA fragment encoding the light-chain variable region of mouse hTAAB using primers including EcoRI and BsiWI restriction sites.

### Human IgG1 heavy-chain constant region (CH1-CH3)

Amino acid sequence (SEQ ID NO: 23) 
ASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKSCDKTH
TCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTI
SKAKGQPREPQVYTLPPSREEMTKNQVSLTCLVKGFYPSDIAVEWESNGQPENNYK
TTPPVLDSDGSFFLYSKLTVDKSRWQQGNVFSCSVMHEALHNHYTQKSLSLSPGK
DNA sequence(SEQ ID NO: 24) 
GCTAGCACCAAGGGCCCATCGGTCTTCCCCCTGGCACCCTCCTCCAAGAGC
ACCTCTGGGGGCACAGCGGCCCTGGGCTGCCTGGTCAAGGACTACTTCCCCGAACC
GGTGACGGTGTCGTGGAACTCAGGCGCCCTGACCAGCGGCGTGCACACCTTCCCGG
CTGTCCTACAGTCCTCAGGACTCTACTCCCTCAGCAGCGTGGTGACCGTGCCCTCC
AGCAGCTTGGGCACCCAGACCTACATCTGCAACGTGAATCACAAGCCCAGCAACAC
CAAGGTGGACAAGAAAGTTGAGCCCAAATCTTGTGACAAAACTCACACATGCCCAC
CGTGCCCAGCACCTGAACTCCTGGGGGGACCGTCAGTCTTCCTCTTCCCCCCAAAA
CCCAAGGACACCCTCATGATCTCCCGGACCCCTGAGGTCACATGCGTGGTGGTGGA
CGTGAGCCACGAAGACCCTGAGGTCAAGTTCAACTGGTACGTGGACGGCGTGGAGG
TGCATAATGCCAAGACAAAGCCGCGGGAGGAGCAGTACAACAGCACGTACCGTGTG
GTCAGCGTCCTCACCGTCCTGCACCAGGACTGGCTGAATGGCAAGGAGTACAAGTG
CAAGGTCTCCAACAAAGCCCTCCCAGCCCCCATCGAGAAAACCATCTCCAAAGCCA
AAGGGCAGCCCCGAGAACCACAGGTGTACACCCTGCCCCCATCCCGGGAGGAGATG
ACCAAGAACCAGGTCAGCCTGACCTGCCTGGTCAAAGGCTTCTATCCCAGCGACAT
CGCCGTGGAGTGGGAGAGCAATGGGCAGCCGGAGAACAACTACAAGACCACGCCTC
CCGTGCTGGACTCCGACGGCTCCTTCTTCCTCTACAGCAAGCTCACCGTGGACAAG
AGCAGGTGGCAGCAGGGGAACGTCTTCTCATGCTCCGTGATGCATGAGGCTCTGCA
 CAACCACTACACGCAGAAGAGCCTCTCCCTGTCTCCGGGTAAA
Human IgG2 heavy-chain constant region (CH1-CH3) Amino acid sequence (SEQ ID NO: 25) 
ASTKGPSVFPLAPCSRSTSESTAALGCLVKDYFPEPVTVSWNSGALTSGVH
TFPAVLQSSGLYSLSSVVTVPSSNFGTQTYTCNVDHKPSNTKVDKTVERKCCVECP
PCPAPPVAGPSVFLFPPKPKDTLMISRTPEVTCVVVDVSHEDPEVQFNWYVDGVEV
HNAKTKPREEQFNSTFRVVSVLTVVHQDWLNGKEYKCKVSNKGLPAPIEKTISKTK
GQPREPQVYTLPPSREEMTKNQVSLTCLVKGFYPSDIAVEWESNGQPENNYKTTPP
MLDSDGSFFLYSKLTVDKSRWQQGNVFSCSVMHEALHNHYTQKSLSLSPGK
DNA sequence (SEQ ID NO: 26) 
GCTAGCACCAAGGGCCCATCGGTCTTCCCCCTGGCGCCCTGCTCCAGGAGC
ACCTCCGAGAGCACAGCGGCCCTGGGCTGCCTGGTCAAGGACTACTTCCCCGAACC
GGTGACGGTGTCGTGGAACTCAGGCGCTCTGACCAGCGGCGTGCACACCTTCCCAG
CTGTCCTACAGTCCTCAGGACTCTACTCCCTCAGCAGCGTGGTGACCGTGCCCTCC
AGCAACTTCGGCACCCAGACCTACACCTGCAACGTAGATCACAAGCCCAGCAACAC
CAAGGTGGACAAGACAGTTGAGCGCAAATGTTGTGTCGAGTGCCCACCGTGCCCAG
CACCACCTGTGGCAGGACCGTCAGTCTTCCTCTTCCCCCCAAAACCCAAGGACACC
CTCATGATCTCCCGGACCCCTGAGGTCACGTGCGTGGTGGTGGACGTGAGCCACGA
AGACCCCGAGGTCCAGTTCAACTGGTACGTGGACGGCGTGGAGGTGCATAATGCCA
AGACAAAGCCACGGGAGGAGCAGTTCAACAGCACGTTCCGTGTGGTCAGCGTCCTC
ACCGTTGTGCACCAGGACTGGCTGAACGGCAAGGAGTACAAGTGCAAGGTCTCCAA
CAAAGGCCTCCCAGCCCCCATCGAGAAAACCATCTCCAAAACCAAAGGGCAGCCCC
GAGAACCACAGGTGTACACCCTGCCCCCATCCCGGGAGGAGATGACCAAGAACCAG
GTCAGCCTGACCTGCCTGGTCAAAGGCTTCTACCCCAGCGACATCGCCGTGGAGTG
 
GGAGAGCAATGGGCAGCCGGAGAACAACTACAAGACCACGCCTCCCATGCTGGACT
CCGACGGCTCCTTCTTCCTCTACAGCAAGCTCACCGTGGACAAGAGCAGGTGGCAG
CAGGGGAACGTCTTCTCATGCTCCGTGATGCATGAGGCTCTGCACAACCACTACAC
GCAGAAGAGCCTCTCCCTGTCTCCGGGTAAA
Human IgG4 heavy-chain constant region (CH1-CH3) Amino acid sequence (SEQ ID NO: 27) 
ASTKGPSVFPLAPCSRSTSESTAALGCLVKDYFPEPVTVSWNSGALTSGVH
TFPAVLQSSGLYSLSSVVTVPSSSLGTKTYTCNVDHKPSNTKVDKRVESKYGPPCP
SCPAPEFLGGPSVFLFPPKPKDTLMISRTPEVTCVVVDVSQEDPEVQFNWYVDGVE
VHNAKTKPREEQFNSTYRVVSVLTVLHQDWLNGKEYKCKVSNKGLPSSIEKTISKA
KGQPREPQVYTLPPSQEEMTKNQVSLTCLVKGFYPSDIAVEWESNGQPENNYKTTP
PVLDSDGSFFLYSRLTVDKSRWQEGNVFSCSVMHEALHNHYTQKSLSLSLGK
DNA sequence (SEQ ID NO: 28)

GCTAGCACCAAGGGCCCATCGGTCTTCCCCCTGGCGCCCTGCTCCAGGAGC
ACCTCCGAGAGCACAGCCGCCCTGGGCTGCCTGGTCAAGGACTACTTCCCCGAACC
GGTGACGGTGTCGTGGAACTCAGGCGCCCTGACCAGCGGCGTGCACACCTTCCCGG
CTGTCCTACAGTCCTCAGGACTCTACTCCCTCAGCAGCGTGGTGACCGTGCCCTCC
AGCAGCTTGGGCACGAAGACCTACACCTGCAACGTAGATCACAAGCCCAGCAACAC
CAAGGTGGACAAGAGAGTTGAGTCCAAATATGGTCCCCCATGCCCATCATGCCCAG
CACCTGAGTTCCTGGGGGGACCATCAGTCTTCCTGTTCCCCCCAAAACCCAAGGAC
ACTCTCATGATCTCCCGGACCCCTGAGGTCACGTGCGTGGTGGTGGACGTGAGCCA
GGAAGACCCCGAGGTCCAGTTCAACTGGTACGTGGATGGCGTGGAGGTGCATAATG
CCAAGACAAAGCCGCGGGAGGAGCAGTTCAACAGCACGTACCGTGTGGTCAGCGTC
CTCACCGTCCTGCACCAGGACTGGCTGAACGGCAAGGAGTACAAGTGCAAGGTCTC
CAACAAAGGCCTCCCGTCCTCCATCGAGAAAACCATCTCCAAAGCCAAAGGGCAGC

CCCGAGAGCCACAGGTGTACACCCTGCCCCCATCCCAGGAGGAGATGACCAAGAAC

CAGGTCAGCCTGACCTGCCTGGTCAAAGGCTTCTACCCCAGCGACATCGCCGTGGA

GTGGGAGAGCAATGGGCAGCCGGAGAACAACTACAAGACCACGCCTCCCGTGCTGG

ACTCCGACGGCTCCTTCTTCCTCTACAGCAGGCTCACCGTGGACAAGAGCAGGTGG

CAGGAGGGGAATGTCTTCTCATGCTCCGTGATGCATGAGGCTCTGCACAACCACTA

CACACAGAAGAGCCTCTCCCTGTCTCTGGGTAAA

Next, the PCR product was subcloned into a modified pcDNA3.3-TOPO vector expressing the human kappa light-chain constant region.

### Human kappa light-chain constant region (GenBank accession number: AAA58989.1)

Amino acid sequence (SEQ ID NO: 29) 
TVAAPSVFIFPPSDEQLKSGTASVVCLLNNFYPREAKVQWKVDNALQSGNS
QESVTEQDSKDSTYSLSSTLTLSKADYEKHKVYACEVTHQGLSSPVTKSFNRGEC
**DNA sequence** (SEQ ID NO: 30)

ACGGTGGCTGCACCATCTGTCTTCATCTTCCCGCCATCTGATGAGCAGTTG
AAATCTGGAACTGCCTCTGTTGTGTGCCTGCTGAATAACTTCTATCCCAGAGAGGC
CAAAGTACAGTGGAAGGTGGATAACGCCCTCCAATCGGGTAACTCCCAGGAGAGTG
TCACAGAGCAGGACAGCAAGGACAGCACCTACAGCCTCAGCAGCACCCTGACGCTG
AGCAAAGCAGACTACGAGAAACACAAAGTCTACGCCTGCGAAGTCACCCATCAGGG
CCTGAGCTCGCCCGTCACAAAGAGCTTCAACAGGGGAGAGTGC

Chimeric antibodies were produced using the Expi293 expression system (ThermoFisher). Expi293F cells were co-transfected with heavy- and light-chain expression vectors using the ExpiFectamine 293 transfection kit (A14524, ThermoFisher), after which the transfected cells were cultured in Expi293 expression medium for 5 days. The culture supernatant was filtered through a 0.45-um filter, and antibodies were purified using an AKTA purification device (GE Healthcare) equipped with a HiTrap MabSelect SuRe column (11003494, GE Healthcare).

In order to delineate the epitope for hTAAB binding, the minimal domain of Tie2 for hTAAB binding was identified. Since hTie2 Ig3-Fn3 was used to immunize mice for antibody production, three recombinant proteins including Ig3-Fn3, Fn1-3, or Fn2-3 of hTie2 were produced (FIG. 1, Tie2 structure). Size-exclusion chromatography (SEC) was used to test binding to recombinant chimeric hTAAB Fab constructed from fusion of the hTAAB heavy-chain variable region to the human γ1 constant region and of the light-chain variable region to the human κ constant region. The chromatographic profile of chimeric hTAAB Fab alone showed a single peak that shifted forward when mixing chimeric hTAAB Fab with hTie2 Ig3-Fn3, Fn1-3, or Fn2-3 (FIG. 2a). Also, chimeric hTAAB Fab and the hTie2 domains were co-eluted. These results show that hTie2 Fn2-3 is sufficient for hTAAB binding and that hTie2 Fn2-3 and chimeric hTAAB Fab are able to form a stable complex (FIG. 2a, yellow).

| hTAAB Fab | Sequence | No. |
|---|---|---|
| hTAAB Mouse γl constant region | AKTTPPSVYPLAPGSAAQTNSMVTLGCLVKGYFPEPVTVTWN SGSLSSGVHTFPAVLQSDLYTLSSSVTVPSSPRPSETVTCNVAH PASSTKVDKKIVPRDCG | 3 |
| hTAAB Mouse κ constant region | RADAAPTVSIFPPSSEQLTSGGASVVCFLNNFYPKDINVKWKI DGSERQNGVLNSWTDQDSKDSTYSMSSTLTLTKDEYERHNSY TCEATHKTSTSPIVKSFNRNEC | 4 |

The crystal structure of the hTie2 Fn2-3/chimeric hTAAB Fab complex at 2.1 Å resolution (Table 1) was determined by molecular replacement using durvalumab Fab (PDB: 5X8M) and Tie2 Fn2-3 structures (PDB: 5MYA chain B) as search models. The crystallographic asymmetric unit contained a 2-fold symmetric heterotetrameric hTie2 Fn2-3/chimeric hTAAB Fab complex (2:2 stoichiometry).

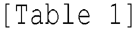

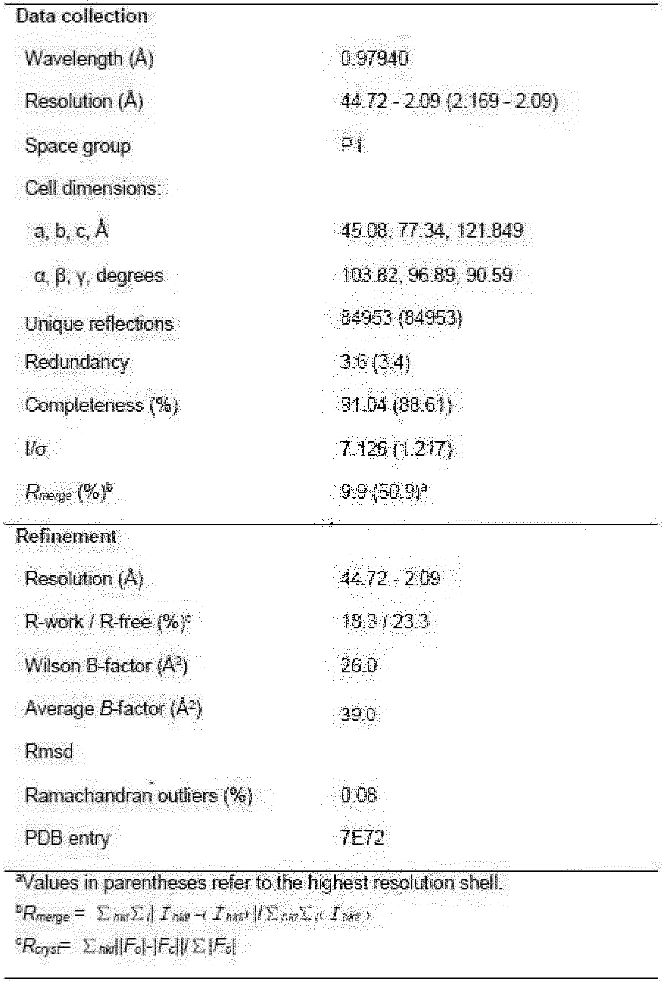

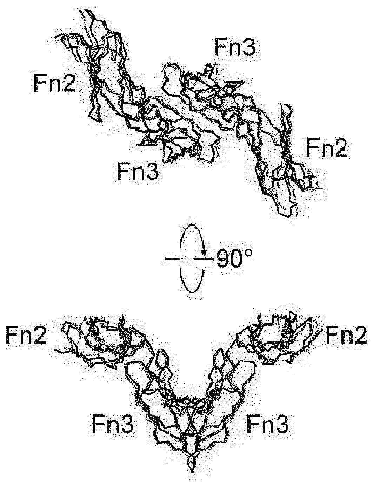

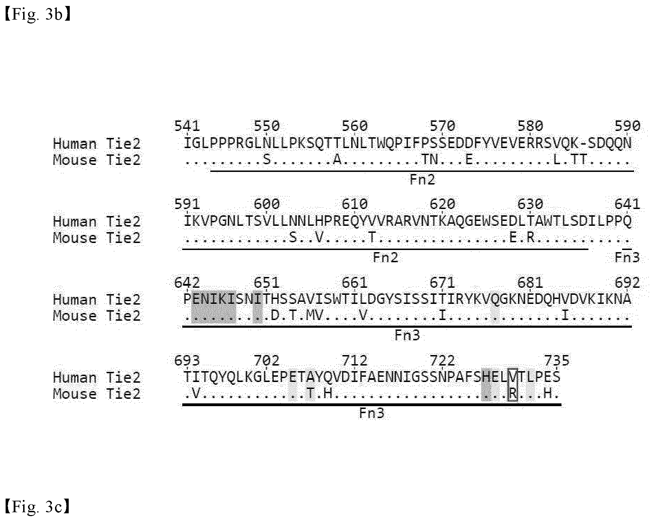

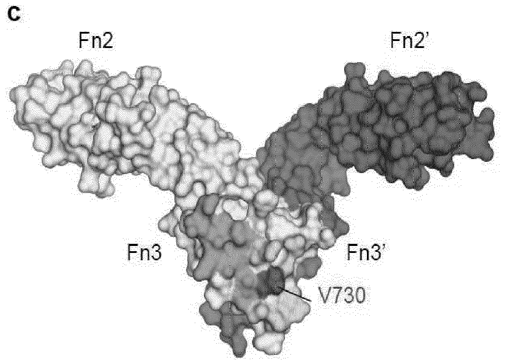

Two chimeric hTAAB Fabs bound to the lateral side of each hTie2 Fn3 domain at a tilt of approximately 15° relative to the plane perpendicular to the 2-fold axis (FIG. 3a, right). This crystal structure revealed two major epitopes of the Fn3 domain for hTAAB binding, colored in dark orange for heavy-chain binding and light orange for light-chain binding (FIGs. 3b and c, top). Two hTie2 Fn2-3 monomers in the heterotetrameric hTie2 Fn2-3/chimeric hTAAB Fab complex were found to share the same overall structure and to be similar to the previously reported crystal structures of Tie2 Fn2-3 (PDB: 5MYB; red) and Fn1-3 (PDB: 5UTK; green), indicating a rigid linkage between Fn2 and Fn3 (FIG. 2b; Cα root mean squared deviation was approximately 1.050 Å between hTie2 Fn2-3 monomers, 1.506 Å with 5MYB, and 1.999 Å with 5UTK). Of note, the configuration and interface between hTie2 dimers in the hTie2 Fn2-3/chimeric hTAAB Fab complex were also consistent with those found in previous studies (5MYB and Dimer2 model of 5UTK) (FIGs. 2c and d).

These results show that hTAAB binding did not cause a change in conformation of the hTie2 homodimer. Four domains of chimeric hTAAB Fab (heavy-chain variable region (VH), heavy-chain constant region (CH), light-chain variable region (VL), and light-chain constant region (CL)) adopted a typical Ig domain fold composed of a pair of β sheets. Heavy-chain CDRs (HCDR1, HCDR2, and HCDR3) and light-chain CDRs (LCDR1, LCDR2, and LCDR3) were involved in contact with the hTie2 Fn3 domain (FIG. 3c, bottom). In addition to six CDRs, light-chain framework region (FR) residues R46, Y49, S53, and R66 also interacted with the hTie2 Fn3 domain (FIG. 3d). Therefore, the findings for the crystal structure revealed that two hTAAB Fabs bind to the lateral sides of the V-shaped Tie2 dimer, particularly to the Fn3 domain.

### Example 3. Interaction of Tie2 Fn3 with hTAAB and homotypic Fn3 interaction

Chimeric hTAAB Fab binds to hTie2 Fn3 through a large interaction interface with a total buried surface area of 897.5 Å² composed of about 57% heavy-chain contacts (512.8 Å²; dark orange) and 43% light-chain contacts (384.7 Å²; light orange) (FIG. 3c). The binding interfaces between chimeric hTAAB Fab and hTie2 Fn3 may be divided into three regions: A, B, and C regions (FIGs. 4a, b, and 5) .

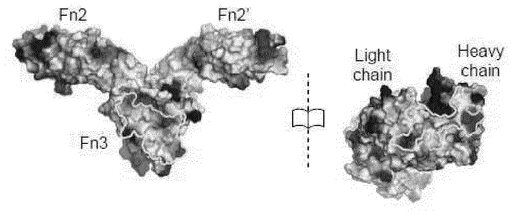

The interactions of the A region are mainly mediated by ionic interactions and hydrogen bonds between the hTAAB heavy chain (HCDR1 and HCDR2) and the hTie2 Fn3 domain. Residues on hTAAB HCDR1 (S28, T30, S31, and W33) and HCDR2 (H52, D55, and E57) form multiple interactions with residues on hTie2 Fn3 βA [E643, N644, I645 (main chain), K646, I647 (main chain)] and βG (H727) (FIG. 4a, top left and bottom left, and FIG. 4c).

In the B region, HCDR3, LCDR1, LCDR2, and LCDR3 of hTAAB cover a wide surface of hTie2 Fn3 by hydrophobic interactions, with a total buried surface area of 413.5 Å²A. Residues I647, I650, A707, V730, and L732 of hTie2 Fn3 form a network of hydrophobic core interactions with residues L100 and Y101 on HCDR3 and I31, Y49, A50, Y91, and A92 on LCDRs (FIG. 4a, bottom left, and FIG. 4d). These hydrophobic interactions are stabilized by neighboring C-region interactions involving hydrogen bonds and electrostatic interactions between hTie2 Fn3 residues (Q677, E705, and E728) and hTAAB light-chain residues (S53, R66, and R46) (FIG. 4a, bottom center, and FIG. 4c).

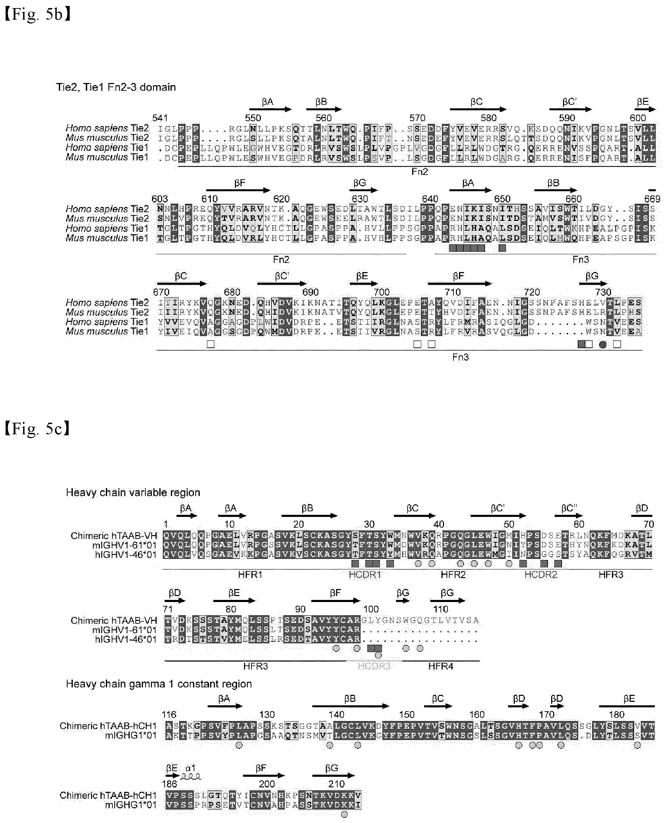

Interestingly, hTAAB light-chain residues involved in the C-region interactions are located on the β-strands of FRs (R46 on βC', S53 on βC', and R66 on βD), rather than the CDR loop. Most hTie2 Fn3 residues involved in hTAAB interactions are highly conserved among various species (FIG. 5a), but hydrophobic valine (V730) of hTie2 at the center of the hTAAB binding interface is substituted with a long charged side chain (arginine) in mouse and rat (FIGs. 3b and 5a), which explains why hTAAB cannot bind to mouse Tie2 (FIG. 1e, right). However, all epitopes of hTAAB are conserved between human and monkey Tie2, suggesting that hTAAB cross-reacts with monkey Tie2 (FIG. 5a). Also, hTAAB is unlikely to bind to Tie1 due to low sequence similarity between Fn2-3 domains of Tie2 and Tie1, especially residues involved in hTAAB interactions (FIG. 5b).

Consistent with previous Tie2 apo-structures (5MYB and Dimer2 model of 5UTK), the Fn3-Fn3' interface is composed of hydrogen bonds between main-chain atoms of homotypic Fn3 βC' (D682-K690), forming a continuous antiparallel β-sheet (FIG. 4a, right). The dimeric interface is further stabilized by hydrophobic interactions involving V685 and V687, as well as by reciprocal electrostatic interactions, especially D682-N691', D682'-N691, Y697-Q683', Y697'-Q683, K700-E703', and K700'-E703 (FIG. 4a, bottom right). The wide buried interface (704 Å²) between Fn3-Fn3' yields low solvation free energy of -4.7 kcal/mol (Pisa server analysis) and consistent V-shaped configuration of the Tie2 homodimer regardless of the presence of hTAAB, indicating that specific interactions between homotypic membrane-proximal Fn3 domains contribute to ligand-independent Tie2 homodimer formation.

Crystal structures and functional analysis using Fn2 mutants have suggested that Fn2 interactions are essential for lateral clustering of preformed Tie2 dimers. Surprisingly, even when the structure contained the Tie2 Fn2-3/hTAAB Fab complex and the space group for the protein crystal was different between two structures, the same interaction was observed between Fn2 domains of neighboring homodimers in the crystal packing (FIGs. 12a and b). However, Fab and Fab* bound to Tie2 Fn3 in the crystal packing lattice are almost parallel to each other (* is used to indicate domains or residues in the symmetric mate of the Tie2 Fn2-3/hTAAB Fab complex), thus deviating from the possible angles (115-148°) between two Fab arms of one IgG1. Therefore, it is unlikely that hTAAB IgG1 is involved in Fn2-mediated lateral clustering of preformed Tie2 dimers, but this type of Tie2 clustering may occur in higher-order Angpt1.

### Example 4. Induction of polygonal assembly of Tie2 dimers by hTAAB IgG binding

Human umbilical vein endothelial cells (HUVECs; C2519A, Lonza) were maintained in EGM-2 endothelial proliferation medium (CC-3162; Lonza) and cultured at 37°C and 5% CO₂ in a humidified incubator. Expi293F cells (A14527, ThermoFisher) were maintained in Expi293 expression medium (A1435102, ThermoFisher) and cultured at 37°C and 8% CO₂ in a humidified shaking incubator.

Multimeric Angpt1 or COMP-Angpt1 is able to induce higher-order clustering of Tie2, which is key for activating Tie2 and downstream signaling thereof in ECs for vascular stabilization. The level of phosphorylation of Akt, which is a major downstream signaling protein of the Tie2 receptor, was measured using immunoblotting technology. Interestingly, not only hTAAB Fab but also hTAAB IgG1 induced concentration-dependent activation of Tie2 and Akt in primarily cultured HUVECs (FIGs. 6a and 6b). This result implied that binding of hTAAB Fab to Tie2 itself was sufficient to induce Tie2 clustering and activation. Briefly, hTAAB IgG1 is capable of facilitating Tie2 clustering through bivalent Fab arms. However, the distance between two C-termini of Fab heavy chains in the heterotetrameric hTie2 Fn2-3/chimeric hTAAB Fab complex is greater than 170 Å (FIG. 3a), suggesting that the two Fabs binding to the homotypic Tie2 dimer are derived from two different IgG molecules. In contrast to the crystal structure of the heterotetrameric hTie2 Fn2-3/chimeric hTAAB Fab complex, SEC-multiangle light scattering (MALS) analysis revealed that hTie2 Fn2-3 and hTie2 ECD were monomeric in solution. Also, hTAAB binding to hTie2 Fn2-3 or hTie2 ECD was incapable of inducing oligomerization (FIG. 6c). The present data and previous reports were contradictory regarding the multimeric status of recombinant Tie2 ectodomain in solution. However, ligand-independent Tie2 dimerization may occur in the cell membrane under physiological conditions through spatial constraints imposed by full-length Tie2. This dimerization may occur particularly through the YIA sequence between catalytic and activation loops of the intracellular kinase domain, with the homotypic Fn3-Fn3' interactions shown in the crystal structure.

In order to mimic ligand-independent Tie2 dimers in a physiological cell membrane, a constitutive Tie2 dimer was artificially produced. The two residues D682 and N691 involved in the homotypic Fn3-Fn3' interaction were mutated to cysteines (D682C and N691C) to introduce a disulfide bond (FIGs. 6d and e). Then, the purified hTie2 Ig3-Fn3 D682C/N691C dimer was incubated with hTAAB IgG1 at a 1:1 ratio to investigate how full-length hTAAB IgG1 induces clustering of preformed Tie2 dimers. After removal of aggregates by SEC, the structural arrangement of the resulting complex (peak of the shoulder) was examined using negative-stain electron microscopy (EM) (FIGs. 6f and 7). Surprisingly, 2D classification of negative-stain EM particles showed that full-length hTAAB IgG1 cross-linked to tetragonal, pentagonal, and hexagonal higher-order assemblies of hTie2 Ig3-Fn3 D682C/N691C dimers (FIG. 6g). These polygonal assemblies are characterized by an Fc domain of IgG1 at each vertex and one Tie2 dimer linking two IgG1s by binding to one of the Fab arms. Although the Ig3 domain appeared diffuse in 2D averages of negative-stain EM due to the flexibility of loop between Ig3 and Fn1 domains, the rigid Fn1-3 domain of the hTie2 Ig3-Fn3 D682C/N691C dimer across the side of the polygon was distinct. Based on observations from negative-stain EM and crystal structure of the hTie2 Fn2-3/chimeric hTAAB Fab complex, a 3D model of pentagonal assemblies (5-to-5 hTAAB IgGs and hTie2 Ig3-Fn3 D682C/N691C dimers) may be generated (FIG. 6h).

### Example 5. Ligand-independent Tie2 dimerization essential for hTAAB-mediated Tie2 clustering and activation

The biological relevance and significance of Tie2/hTAAB polygonal assemblies in the cell membrane were evaluated. The Tie2 dimer (D682C/N691C) was used to construct a Tie2 monomer (V685D/V687D/K700E) along with a full-length Tie2-GFP construct by disrupting the Fn3-Fn3' dimeric interface (FIG. 8a). This construct was used to transiently transfect HEK293T cells that do not endogenously express Tie2. After confirming adequate plasma membrane expression of full-length Tie2-GFP wild-type (WT) and constitutive dimeric and monomeric Tie2 mutants in the cells (FIG. 8b), clustering of GFP-tagged Tie2 WT and mutants was monitored. Live-cell imaging showed that clusters of WT Tie2 and Tie2 dimers were formed at the cell surface after addition of hTAAB or COMP-Angpt1, whereas Tie2 monomers failed to form clusters (FIG. 8b). Next, the effect of hTAAB IgG1 or COMP-Angpt1 on Tie2 activation was measured by monitoring phosphorylation of Tie2 and Akt under the same conditions. Although disruption of the Fn3-Fn3 dimeric interface (constitutive Tie2 monomers) abolished phosphorylation of Tie2 and Akt induced by hTAAB and COMP-Angpt1, WT Tie2 and dimeric Tie2 were similarly activated by both Tie2 agonists (FIGs. 8c and d). These results show that WT Tie2 exists as a homotypic dimer in the plasma membrane and that ligand-independent homotypic Tie2 dimerization, which is attributable to intermolecular interactions between Fn3 domains, is essential for hTAAB-mediated Tie2 clustering and activation.

### Example 6. Activation of Tie2 and downstream signaling thereof in HUVECs by humanized TAAB

### 6.1 Production of humanized antibody

The Fv region of mouse hTAAB was humanized based on the Tie2 Fn2-3/chimeric hTAAB Fab complex structure and the model structure of IGHV1-46*01 and IGKV1-17*01 complexes.

The mouse hTAAB Fv sequence was compared with that of human germline genes using the IMGT/DomainGapAlign online tool from the International ImmunoGeneTics Information System (IMGT) (http://www.imgt.org). IGHV1-46*01 and IGKV1-17*01, which exhibited the highest sequence identity to heavy- and light-chain variable regions of mouse hTAAB, respectively, were selected as human framework (FR) donors. The CDRs (defined by IMGT numbering) of IGHV1-46*01 and IGKV1-17*01 were replaced with the counterparts in mouse hTAAB, producing hzTAAB-H1 and hzTAAB-L1. The selected residues critical for maintaining VH-VL pairing, CDR conformation, and affinity for Tie2 Fn3 were further substituted on hzTAAB-H1 and hzTAAB-L1, producing hzTAAB-H2 and hzTAAB-L2. The resulting humanized Fv genes (hzTAAB-H1, hzTAAB-H2, hzTAAB-L1, and hzTAAB-L2) were synthesized (Bioneer) and optimized for expression in mammalian cells. The human immunoglobulin kappa light-chain constant region (GenBank accession number: AAA58989.1) and the human immunoglobulin gamma 1 heavy-chain constant region (GenBank accession number: AWK57454.1) were first cloned into pOptiVEC-TOPO and pcDNA3.3-TOPO vectors, into which the synthesized variable regions of humanized light and heavy chains (hzTAAB-H1, hzTAAB-H2, hzTAAB-L1, or hzTAAB-L2) were subsequently cloned.

### Human kappa light-chain constant region (GenBank accession number: AAA58989.1)

**Amino acid sequence** (SEQ ID NO: 31) 
TVAAPSVFIFPPSDEQLKSGTASVVCLLNNFYPREAKVQWKVDNALQSGNS
QESVTEQDSKDSTYSLSSTLTLSKADYEKHKVYACEVTHQGLSSPVTKSFNRGEC
**DNA sequence** (SEQ ID NO: 32)

ACGGTGGCTGCACCATCTGTCTTCATCTTCCCGCCATCTGATGAGCAGTTG
AAATCTGGAACTGCCTCTGTTGTGTGCCTGCTGAATAACTTCTATCCCAGAGAGGC
CAAAGTACAGTGGAAGGTGGATAACGCCCTCCAATCGGGTAACTCCCAGGAGAGTG
TCACAGAGCAGGACAGCAAGGACAGCACCTACAGCCTCAGCAGCACCCTGACGCTG
AGCAAAGCAGACTACGAGAAACACAAAGTCTACGCCTGCGAAGTCACCCATCAGGG
CCTGAGCTCGCCCGTCACAAAGAGCTTCAACAGGGGAGAGTGC

### Human IgG1 heavy-chain constant region (CH1-CH3) (GenBank accession number: AWK57454.1)

**Amino acid sequence** (SEQ ID NO: 33) 
ASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKSCDKTH
TCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTI
SKAKGQPREPQVYTLPPSREEMTKNQVSLTCLVKGFYPSDIAVEWESNGQPENNYK
TTPPVLDSDGSFFLYSKLTVDKSRWQQGNVFSCSVMHEALHNHYTQKSLSLSPGK
**DNA sequence** (SEQ ID NO: 34)

GCTAGCACCAAGGGCCCATCGGTCTTCCCCCTGGCACCCTCCTCCAAGAGC
ACCTCTGGGGGCACAGCGGCCCTGGGCTGCCTGGTCAAGGACTACTTCCCCGAACC
GGTGACGGTGTCGTGGAACTCAGGCGCCCTGACCAGCGGCGTGCACACCTTCCCGG

CTGTCCTACAGTCCTCAGGACTCTACTCCCTCAGCAGCGTGGTGACCGTGCCCTCC
AGCAGCTTGGGCACCCAGACCTACATCTGCAACGTGAATCACAAGCCCAGCAACAC
CAAGGTGGACAAGAAAGTTGAGCCCAAATCTTGTGACAAAACTCACACATGCCCAC
CGTGCCCAGCACCTGAACTCCTGGGGGGACCGTCAGTCTTCCTCTTCCCCCCAAAA
CCCAAGGACACCCTCATGATCTCCCGGACCCCTGAGGTCACATGCGTGGTGGTGGA
CGTGAGCCACGAAGACCCTGAGGTCAAGTTCAACTGGTACGTGGACGGCGTGGAGG
TGCATAATGCCAAGACAAAGCCGCGGGAGGAGCAGTACAACAGCACGTACCGTGTG
GTCAGCGTCCTCACCGTCCTGCACCAGGACTGGCTGAATGGCAAGGAGTACAAGTG
CAAGGTCTCCAACAAAGCCCTCCCAGCCCCCATCGAGAAAACCATCTCCAAAGCCA
AAGGGCAGCCCCGAGAACCACAGGTGTACACCCTGCCCCCATCCCGGGAGGAGATG
ACCAAGAACCAGGTCAGCCTGACCTGCCTGGTCAAAGGCTTCTATCCCAGCGACAT
CGCCGTGGAGTGGGAGAGCAATGGGCAGCCGGAGAACAACTACAAGACCACGCCTC
CCGTGCTGGACTCCGACGGCTCCTTCTTCCTCTACAGCAAGCTCACCGTGGACAAG
AGCAGGTGGCAGCAGGGGAACGTCTTCTCATGCTCCGTGATGCATGAGGCTCTGCA
CAACCACTACACGCAGAAGAGCCTCTCCCTGTCTCCGGGTAAA

Expi293F cells were transfected with plasmids containing humanized light and heavy chains using an ExpiFectamine 293 transfection kit (A14524, ThermoFisher). The cells were cultured for 5 days at 37°C and 8% CO₂ in a shaking incubator (orbital shaker, 125 rpm). The obtained culture supernatant was centrifuged to remove cells, and antibodies were isolated by affinity chromatography on a ProA column (Amicogen) equilibrated with PBS. The bound antibodies were eluted with 0.1 M glycine-HCl, pH 2.7, and neutralized with 1 M Tris-Cl, pH 9.0. The eluted antibodies were further purified by size-exclusion chromatography using a Superdex 200 Gain 10/300 GL column (28-9909-44, GE Healthcare) equilibrated with PBS. Antibody purity was evaluated using reducing and nonreducing SDS-PAGE, and purified antibodies were dispensed and stored at -80°C.

### 6.2 Homology modeling of humanized Tie2-activating antibody

SWISS-MODEL homology modeling was performed with sequences of human germline IGHV1-46*01 and IGKV1-17*01 grafted with hTAAB CDRs using the structure of hTAAB Fv in the Tie2 Fn2-3/chimeric hTAAB Fab complex structure as a template. The resulting model with the highest QMEAN-Z (Qualitative Model Energy ANalysis-Z) score (-0.37) was aligned to the hTAAB Fv structure for further structure-based humanization.

| Antibody | sequence (VH) | No. | Sequence (VL) | No. |
|---|---|---|---|---|
| hzTAAB-H1L1 | (Protein sequence) | 5 | (Protein sequence) | 6 |
| | QVQLVQSGAEVKKPGASVKVSCKASGYSF TSYWMHWVRQAPGQGLEWMGIIHPSDSE TRYAQKFQGRVTMTRDTSTSTVYMELSSL RSEDTAVYYCARGLYGNSWGQGTLVTVSS | | DIQMTQSPSSLSASVGDRVTITCRASQDI GISLGWYQQKPGKAPKRLIYATSSLQSG VPSRFSGSGSGTEFTLTISSLQPEDFATY YCLQYASSPYTFGQGTKLEIK | |
| | (Coding nucleotide sequence) | 11 | (Coding nucleotide sequence) | 12 |
| | CAGGTTCAGCTGGTCCAGAGCGGGGCT GAGGTCAAGAAACCAGGTGCATCCGTTA AGGTGTCATGTAAAGCCTCGGGGTACAG TTTCACCAGCTACTGGATGCACTGGGTG AGACAGGCCCCTGGTCAAGGCCTTGAAT GGATGGGAATTATCCATCCCTCCGATTCC GAAACTAGATATGCACAAAAGTTTCAGGG CAGAGTGACTATGACCAGAGACACATCTA CGTCTACCGTCTATATGGAGTTGTCAAGT CTGCGCAGCGAGGACACAGCTGTATACT ATTGCGCCAGGGGACTCTATGGCAATAG CTGGGGACAGGGCACTCTGGTGACAGT GTCTTCA | | GATATCCAGATGACTCAATCTCCTAGCT CCCTCAGCGCTTCAGTGGGCGACAGA GTCACCATTACCTGTCGCGCTTCTCAG GATATCGGGATTAGCCTGGGATGGTAC CAGCAGAAGCCTGGTAAGGCACCCAA ACGGCTGATTTACGCCACATCGAGTCT GCAGTCTGGAGTGCCAAGCAGGTTTTC AGGAAGCGGGTCCGGCACCGAGTTTA CTCTGACAATATCTAGTCTGCAGCCAGA AGACTTCGCCACATACTATTGCTTGCAG TATGCATCCTCACCCTATACGTTCGGCC AAGGAACCAAACTTGAGATCAAG | |
| hzTAAB-H2L1 | (Protein sequence) | 7 | (Protein sequence) | 6 |
| | QVQLVQSGAEVKKPGASVKVSCKASGYSF TSYWMHWVRQAPGQGLEWMGIIHPSDSE TRYAQKFQGRVTMTVDKSTSTVYMELSSL RSEDTAVYYCARGLYGNSWGQGTLVTVSS | | DIQMTQSPSSLSASVGDRVTITCRASQDI GISLGWYQQKPGKAPKRLIYATSSLQSG VPSRFSGSGSGTEFTLTISSLQPEDFATY YCLQYASSPYTFGQGTKLEIK | |
| | (Coding nucleotide sequence) | 13 | (Coding nucleotide sequence) | 12 |
| | CAGGTCCAGCTGGTGCAGAGTGGCGCC GAGGTCAAGAAGCCTGGAGCATCAGTTA AAGTGTCTTGTAAAGCTAGTGGCTACTCG TTTACTTCTTACTGGATGCACTGGGTGAG ACAAGCCCCAGGTCAAGGATTGGAGTGG ATGGGCATTATCCATCCCAGCGACTCCGA AACGAGATATGCTCAGAAGTTCCAGGGG CGGGTGACAATGACCGTCGATAAATCTAC AAGCACCGTGTACATGGAGCTTTCCAGT CTGAGATCCGAAGACACTGCCGTATACTA TTGCGCAAGGGGCCTCTATGGTAATTCAT GGGGACAGGGGACCCTGGTGACAGTTA GCTCC | | GATATCCAGATGACTCAATCTCCTAGCT CCCTCAGCGCTTCAGTGGGCGACAGA GTCACCATTACCTGTCGCGCTTCTCAG GATATCGGGATTAGCCTGGGATGGTAC CAGCAGAAGCCTGGTAAGGCACCCAA ACGGCTGATTTACGCCACATCGAGTCT GCAGTCTGGAGTGCCAAGCAGGTTTTC AGGAAGCGGGTCCGGCACCGAGTTTA CTCTGACAATATCTAGTCTGCAGCCAGA AGACTTCGCCACATACTATTGCTTGCAG TATGCATCCTCACCCTATACGTTCGGCC AAGGAACCAAACTTGAGATCAAG | |

| | | | | |
|---|---|---|---|---|
| hzTAAB-H1L2 | (Protein sequence) | 5 | (Protein sequence) | 8 |
| | QVQLVQSGAEVKKPGASVKVSCKASGYSF TSYWMHWVRQAPGQGLEWMGIIHPSDSE TRYAQKFQGRVTMTRDTSTSTVYMELSSL RSEDTAVYYCARGLYGNSWGQGTLVTVSS | | DIQMTQSPSSLSASVGDRVTITCRASQDI GISLNWLQQKPGKAPKRLIYATSSLDSGV PSRFSGSRSGTEFTLTISSLQPEDFATYY CLQYASSPYTFGQGTKLEIK | |
| | (Coding nucleotide sequence) | 11 | (Coding nucleotide seauencel | 14 |
| | CAGGTTCAGCTGGTCCAGAGCGGGGCT GAGGTCAAGAAACCAGGTGCATCCGTTA AGGTGTCATGTAAAGCCTCGGGGTACAG TTTCACCAGCTACTGGATGCACTGGGTG AGACAGGCCCCTGGTCAAGGCCTTGAAT GGATGGGAATTATCCATCCCTCCGATTCC GAAACTAGATATGCACAAAAGTTTCAGGG CAGAGTGACTATGACCAGAGACACATCTA CGTCTACCGTCTATATGGAGTTGTCAAGT CTGCGCAGCGAGGACACAGCTGTATACT ATTGCGCCAGGGGACTCTATGGCAATAG CTGGGGACAGGGCACTCTGGTGACAGT GTCTTCA | | GACATCCAAATGACTCAGAGCCCCTCC AGTCTGTCGGCATCTGTGGGAGATCGC GTCACCATTACATGCCGGGCCAGCCAG GACATCGGCATTAGTTTGAACTGGCTT CAGCAGAAGCCTGGGAAAGCCCCAAA AAGACTCATATACGCTACTTCTTCCCTC GACTCTGGCGTGCCCTCAAGATTTAGC GGAAGCAGGTCAGGCACTGAGTTCAC CCTGACAATTAGCTCCCTGCAACCAGA AGATTTCGCTACATACTATTGTCTGCAG TACGCCTCATCCCCTTATACCTTTGGTC AGGGGACTAAGCTGGAGATCAAG | |
| hzTAAB-H2L2 | (Protein sequence) | 7 | (Protein sequence) | 8 |
| | [?] QVQLVQSGAEVKKPGASVKVSCKASGYSF TSYWMHWVRQAPGQGLEWMGIIHPSDSE TRYAQKFQGRVTMTVDKSTSTVYMELSSL RSEDTAVYYCARGLYGNSWGQGTLVTVSS | | DIQMTQSPSSLSASVGDRVTITCRASQDI GISLNWLQQKPGKAPKRLIYATSSLDSGV PSRFSGSRSGTEFTLTISSLQPEDFATYY CLQYASSPYTFGQGTKLEIK | |
| | Coding nucleotide sequence) | 13 | (Coding nucleotide sequence) | 14 |
| | CAGGTCCAGCTGGTGCAGAGTGGCGCC GAGGTCAAGAAGCCTGGAGCATCAGTTA AAGTGTCTTGTAAAGCTAGTGGCTACTCG TTTACTTCTTACTGGATGCACTGGGTGAG ACAAGCCCCAGGTCAAGGATTGGAGTGG ATGGGCATTATCCATCCCAGCGACTCCGA AACGAGATATGCTCAGAAGTTCCAGGGG CGGGTGACAATGACCGTCGATAAATCTAC AAGCACCGTGTACATGGAGCTTTCCAGT CTGAGATCCGAAGACACTGCCGTATACTA TTGCGCAAGGGGCCTCTATGGTAATTCAT GGGGACAGGGGACCCTGGTGACAGTTA GCTCC | | GACATCCAAATGACTCAGAGCCCCTCC AGTCTGTCGGCATCTGTGGGAGATCGC GTCACCATTACATGCCGGGCCAGCCAG GACATCGGCATTAGTTTGAACTGGCTT CAGCAGAAGCCTGGGAAAGCCCCAAA AAGACTCATATACGCTACTTCTTCCCTC GACTCTGGCGTGCCCTCAAGATTTAGC GGAAGCAGGTCAGGCACTGAGTTCAC CCTGACAATTAGCTCCCTGCAACCAGA AGATTTCGCTACATACTATTGTCTGCAG TACGCCTCATCCCCTTATACCTTTGGTC AGGGGACTAAGCTGGAGATCAAG | |

Potent Tie2-agonistic activity of hTAAB is promising in clinical applications for vascular diseases. Therefore, attempts were made to humanize mouse hTAAB while maintaining the ability to bind to and activate Tie2 but minimizing immunogenicity, which is attributable to mouse origin thereof. Due to variation in length, sequence, and the number of disulfide bonds at the hinge between Fab and Fc domains, the hinge flexibility of IgG subclasses is slightly variable, and the Fab-Fab angle and Fab orientation relative to the Fc domain for each subclass are also variable.

Considering that the Fab-Fab angle and Fab orientation of hTAAB may be key determinants of polygonal assembly of Tie2, whether this hinge flexibility affects Tie2 activation was investigated. To this end, chimeric IgG1, IgG2, or IgG4 including the variable region of hTAAB was constructed (FIG. 9a), and Tie2-agonistic activities thereof were compared with those of parental mouse hTAAB. Phosphorylation of Tie2 and Akt correlated with the hinge flexibility of the IgG subclass (IgG1 > IgG4 > IgG2). The IgG2 isotype with the most rigid hinge exhibited the lowest Tie2-agonistic activity (FIGs. 9b and c). These results show that polygonal Tie2 clustering induced by hTAAB is critical for Tie2 activation. Based on these observations, the IgG1 format for hTAAB humanization was chosen.

Using the IMGT/Domain Gap Align tool from IMGT (International ImmunoGeneTics information system) (http://www.imgt.org), CDR boundaries were defined and human germline genes IGHV1-46*01 and IGKV1-17*01 were selected as human FR donors because they showed the highest sequence identities to the heavy-chain variable region (66%) and light-chain variable region (68%) of mouse hTAAB (FIGs. 9d and 5C-D).

Binding kinetics of mouse hTAAB and humanized IgG1 for human Tie2 were measured by surface plasmon resonance (SPR) using a Biacore T200 system equipped with certified-grade CM5 series S sensor chips (BR100399, GE Healthcare). HEPES-buffered saline (0.01 M HEPES and 0.15 M NaCl) containing 3 mM EDTA (ethylenediaminetetraacetic acid) and 0.05% (v/v) P20 detergent (HBS-EP +) was used as reaction and running buffer (BR100669, GE Healthcare). Human Tie2 ECD (produced in-house; residues 23-735 of human Tie2, GenBank accession number: AAH35514.2) was immobilized on the surface of a CM5 sensor chip by amine coupling using 10 mM acetate at a pH of 5.5. Thereafter, mouse hTAAB and humanized IgG1 diluted in HBS-EP + buffer were applied over antigen-immobilized sensor chips at 7 different concentrations (0, 2, 4, 8, 16, 32, and 64 nM) for 300 seconds at a flow rate of 30 µl/min. Analytes bound to the sensor chips were dissociated by washing with HBS-EP + running buffer for 300 seconds. Both association (kₒₙ, M⁻¹s⁻¹) and dissociation (k_{off}, s⁻¹) constants were measured at 300-second intervals. The equilibrium dissociation constant (K_{D}, M) was calculated as the ratio of off-rate to on-rate (k_{off}/kₒₙ). Kinetic parameters were determined with the global fitting function of Biacore Insight Evaluation Software using a 1:1-binding model.

Binding kinetics of mouse hTAAB and humanized IgG1 for mouse Tie2 were measured by BLI (biolayer interferometry) using the Octet system (ForteBio). Analysis was performed at 30°C. After hydration for 10 minutes in kinetics buffer (0.01% endotoxin-free BSA, 0.002% Tween-20, and 0.005% NaN₃ in PBS), mouse hTAAB (10 ug/ml each) was loaded onto anti-mouse IgG Fc capture (AMC) biosensors (ForteBio). Ab-coated sensors were incubated with a 1600 nM solution of mouse Tie2 Ig3-Fn3 (residues 349-737) and thus association curves were recorded for 600 seconds. Dissociation was measured for 600 seconds in kinetics buffer.

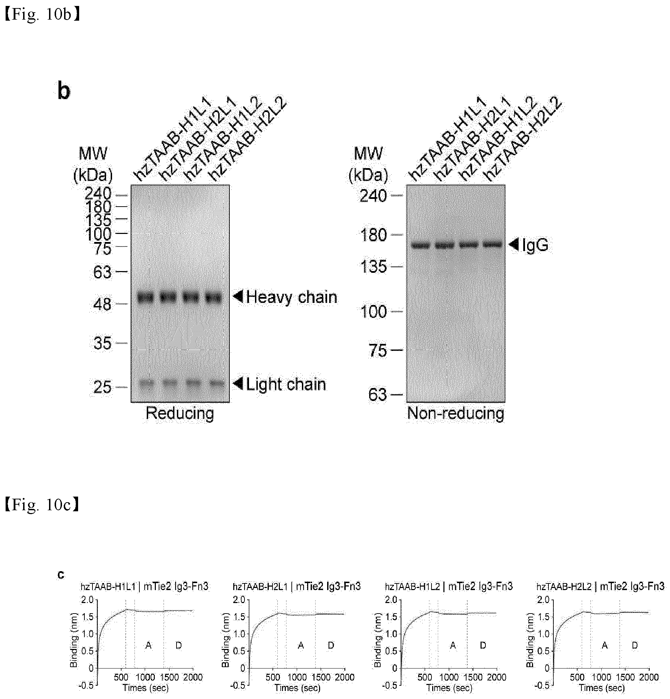

Conventional CDR grafting was performed by simply replacing CDR portions in the selected human germline FR donors with corresponding CDRs of hybridoma mouse antibody hTAAB. In order to maintain the conformation of hTAAB HCDR2, S59 was replaced with a R59 residue flanking the HCDR2 of hTAAB heavy chains in the humanized antibody. The CDR-grafted variable regions of humanized Tie2-activating antibody (hzTAAB) heavy and light chains (hzTAAB-H1 and hzTAAB-L1) were cloned into a pOptiVEC-TOPO vector including the human Ig γ1 heavy-chain constant region (GenBank accession number: AWK57454.1) and a pcDNA3.3-TOPO vector including the human Ig κ light-chain constant region (GenBank accession number: AAA58989.1), respectively. After co-expression of hzTAAB-H1 and hzTAAB-L1 in HEK293F cells, the antibodies were purified to homogeneity as full-length IgG1 by protein A affinity chromatography and SEC (FIGs. 10a and b), and binding kinetics of hzTAAB-H1L1 to hTie2 ECD were evaluated using SPR. hzTAAB-H1L1 binding to hTie2 exhibited much lower affinity and faster dissociation rate (kₒₙ = 2.5×10⁵ M⁻¹s⁻¹; k_{off} = 5.6×10⁻³ s⁻¹; K_{D} = 2.2×10⁻⁸ M) than parental hTAAB (FIG. 9f), indicating that conventional CDR grafting is insufficient to maintain binding affinity of hTAAB to hTie2.

Regarding structure-based humanization of hTAAB, homology modeling of hzTAAB-H1 and hzTAAB-L1 was performed using the Fv (variable fragment) structure of parental hTAAB in the crystal structure as a template. The parental hTAAB Fv structure was placed on the resulting hzTAAB-H1L1Fv model to identify FR residues critical for maintaining VH-VL pairing, CDR conformation, and binding affinity to hTie2 (FIG. 9e). Comparative analysis between the VH-VL interface of hzTAAB-H1L1 and parental hTAAB showed that the hTAAB light-chain residues N34 and L36 (G34 and Y36 in hzTAAB-L1) on LFR2 stabilize Y101 on HCDR3 and decrease steric hindrance with W105 on HFR4. Also, hTAAB light-chain residue D55 (Q55 in hzTAAB-L1) on LFR3 is critical for maintaining the conformation of the LCDR2 hairpin loop by interacting with R46 on LFR2 (f in FIG. 9e). hTAAB light-chain residue R66 (G66 in hzTAAB-L1) on LFR3 is also critical for binding to Tie2 through charge interactions with E705 of hTie2 (g in FIG. 9e). Based thereon, hzTAAB-L2 was produced by back-mutating selected residues of hzTAAB-L1 to the corresponding mouse hTAAB residues (G34N, Y36L, Q55D, and G66R) (FIG. 9d). For heavy chain (hzTAAB-H2), R72 and T74 on HFR3 of hzTAAB-H1 were mutated to parental hTAAB residues V72 and K74, which potentially stabilize the HCDR2 conformation by interacting with P53 and S54 of HCDR2 (h in FIG. 9e).

Following hzTAAB-H1L1, three other hzTAABs (hzTAAB-H1L2, hzTAAB-H2L1, and hzTAAB-H2L2) were produced depending on different combinations of heavy and light chains, all as full-length IgG1s in HEK293F cells (FIGs. 10a and b). After purification, binding kinetics of the obtained antibodies to hTie2 ECD were evaluated using SPR. The binding affinity of hzTAAB-H2L1 (kₒₙ = 2.8×10⁵ M⁻¹s⁻¹; k_{off} = 3.3×10⁻³ s⁻¹; K_{D} = 1.1×10⁻⁸ M) was slightly higher than that of hzTAAB-H1L1 (kₒₙ = 2.5×10⁵ M⁻¹s⁻¹; k_{off} = 5.6×10⁻³ s⁻¹; K_{D} = 2.2×10⁻⁸ M), but the dissociation rate thereof was much higher than that of parental hTAAB (kₒₙ = 1.4×10⁵ M⁻¹s⁻¹; k_{off} = 6.0×10⁻⁴ s⁻¹; K_{D} = 4.2×10⁻⁹ M) (FIG. 9i) . However, back-mutations in the light chain significantly improved the affinities of hzTAAB-H1L2 (kₒₙ = 1.1×10⁵ M⁻¹s⁻¹; k_{off} = 9.6×10⁻⁴ s⁻¹; K_{D} = 8.5×10⁻⁹ M) and hzTAAB H2L2 (kₒₙ = 1.4×10⁵ M⁻¹s⁻¹; k_{off} = 7.3×10⁻⁴ s⁻¹; K_{D} = 5.2×10⁻⁹ M) for hTie2 ECD (FIG. 9i) . The affinities of the obtained humanized antibodies (hzTAAB-H1L2 and hzTAAB-H2L2) were comparable to the affinity of parental hTAAB, and were achieved mainly by a significant reduction in dissociation rate. Also, when comparing the Tie2 binding affinity with the previously reported humanized antibody 3H7 called 3H7H12G4, hzTAAB-H2L2 exhibits 4,700-fold higher affinity than 3H7H12G4, suggesting an improvement in the structure-based hTAAB humanization process (FIG. 9i). Similar to parental hTAAB, none of humanized antibodies bound to mTie2 (FIG. 10c).

| Antibody | Antibody Sequence (VH) | Antibody Sequence (VL) |
|---|---|---|
| 3H7H12G4 | (Protein Sequence) | (Protein Sequence) |
| | [?] QVQLVQSGAEVKKPGASVKV SCKASGYTFTSYWMNWVRQ APGQGLEWMGMIHPSDSETR LNQKFMDRVTMTRDTSTSTV YMELSSLRSEDTAVYYCARGL YGNSWGQGTLVTVSS (SEQ ID NO: 15) | [?] DIQMTQSPSSLSASVGDRVTIT CRASQDIGISLNWLQQEPGKA PKRLIYATSSLDSGVPKRFSGS GSGTEFTLTISSLQPEDFATYY CLQYASSPYTFGQGTKVEIK (SEQ ID NO: 16) |
| | (Coding Nucleotide Sequence) | (Coding Nucleotide Sequence) |
| | CAGGTGCAGCTGGTCCAATC CGGGGCTGAGGTGAAGAAG CCTGGAGCATCAGTGAAAGT TTCATGCAAAGCTAGTGGTT ACACCTTCACCAGCTATTGG ATGAACTGGGTGCGGCAGGC CCCCGGTCAGGGGCTTGAGT GGATGGGCATGATCCACCCA TCCGACTCTGAGACTAGGCT GAACCAGAAGTTTATGGATA GAGTGACCATGACAAGAGAT ACGTCCACTTCTACTGTCTAT ATGGAACTGAGCAGTCTGAG ATCTGAAGACACAGCCGTTT ACTACTGTGCTCGCGGACTC TATGGCAATAGCTGGGGCCA AGGAACATTGGTAACCGTCT CTTCT (SEQ ID NO: 17) | GACATCCAGATGACTCAGTC CCCCTCGAGCCTCTCAGCTT CTGTTGGAGACAGAGTGACA ATTACATGCCGGGCCTCACA GGATATTGGGATCTCCCTGAA CTGGCTGCAACAGGAACCA GGAAAGGCCCCTAAGCGCCT GATATATGCCACATCCTCTCT TGACTCAGGGGTCCCAAAGA GGTTTAGCGGCAGTGGATCA GGTACTGAGTTCACTCTCAC CATCTCTAGCCTGCAGCCTG AGGATTTTGCAACCTACTATT GTTTGCAATACGCTAGTTCCC CCTACACGTTCGGCCAGGGC ACCAAAGTGGAAATCAAA (SEQ ID NO: 18) |

Subsequently, phosphorylation of Tie2 and Akt upon hzTAAB treatment was examined and compared with the phosphorylation after treatment with hTAAB or 3H7H12G4. Consistent with the Tie2 binding affinity, previously developed 3H7H12G4 showed very low Tie2-agonistic activity compared to hTAAB (FIGs. 11a and b). Among four hzTAABs, hzTAAB-H2L2 most potently induced phosphorylation of Tie2 and Akt in HUVECs (FIGs. 11c and d). This is comparable to the Tie2-agonistic activity of hTAAB. Also, when comparing hzTAAB-H2L2 with 3H7H12G4, hzTAAB-H2L2 exhibited excellent Tie2-agonistic activity (FIGs. 11e and f).

Accordingly, hzTAAB-H2L2 potently induces survival, migration, and tube formation of ECs, and Tie2 translocation to cell-cell contact sites and FOXO1 translocation from the nucleus to cytosol, to a similar extent to COMP-Angpt1 and parental hTAAB (FIGs. 11g-i). Taken together, the structure-based humanization strategy successfully retains the binding affinity and Tie2-agonistic activity of parental hTAAB.

### [Industrial Applicability]

The inventors of the present application developed a human Tie2-agonistic antibody hTAAB targeting the Tie2 Fn (membrane proximal fibronectin type III) domain and a humanized antibody thereof. The Tie2/hTAAB complex structure operates in a new mode of Tie2 clustering. It can be confirmed that hTAAB operates in a new mode of Tie2 clustering by forming a Tie2/hTAAB complex structure, and binds specifically to the Tie2 Fn3 domain, connecting Tie2 homodimers into a polygonal assembly. The importance of Fn3-mediated Tie2 homodimerization for Tie2 polygonal assemblies induced by hTAAB can be confirmed and how the Tie2 agonist induces Tie2 clustering and activation can be understood. Also, potential clinical applicability can be confirmed by constructing a humanized antibody based on the structure of hTAAB.

Having described specific parts of the present invention in detail above, it will be obvious to those skilled in the art that these specific descriptions are only preferred embodiments, and the scope of the present invention is not limited thereby. Accordingly, the substantial scope of the present invention will be defined by the appended claims and equivalents thereto.

### [Sequence List Free Text]

An electronic file is attached.

## Claims

1. A Tie2-agonistic antibody or antigen-binding fragment thereof, wherein the antibody binds to an Ig3 Fn3 (membrane proximal fibronectin type III) domain of human Tie2, and by binding of the antibody, homodimeric Tie2 is formed into a polygonal assembly, and thus clustered and activated.

2. The antibody or antigen-binding fragment thereof according to claim 1, wherein the antibody is Fab comprising a heavy-chain variable region (VH) comprising a sequence of SEQ ID NO: 1, a heavy-chain constant region (CH) comprising a sequence of SEQ ID NO: 3, a light-chain variable region (VL) comprising a sequence of SEQ ID NO: 2, and a light-chain constant region (CL) comprising a sequence of SEQ ID NO: 4.

3. The antibody or antigen-binding fragment thereof according to claim 1, comprising:
a heavy-chain variable region comprising an amino acid sequence of SEQ ID NO: 5 or SEQ ID NO: 7; and
a light-chain variable region including an amino acid sequence of SEQ ID NO: 6 or SEQ ID NO: 8.

4. The antibody or antigen-binding fragment thereof according to claim 3, comprising:
a heavy-chain variable region comprising an amino acid sequence of SEQ ID NO: 5 and a light-chain variable region comprising an amino acid sequence of SEQ ID NO: 6;
a heavy-chain variable region comprising an amino acid sequence of SEQ ID NO: 7 and a light-chain variable region comprising an amino acid sequence of SEQ ID NO: 6;
a heavy-chain variable region comprising an amino acid sequence of SEQ ID NO: 5 and a light-chain variable region comprising an amino acid sequence of SEQ ID NO: 8; or
a heavy-chain variable region comprising an amino acid sequence of SEQ ID NO: 7 and a light-chain variable region comprising an amino acid sequence of SEQ ID NO: 8.

5. A nucleic acid encoding the antibody or antigen-binding fragment thereof according to any one of claims 1 to 4.

6. The nucleic acid according to claim 5, comprising a sequence selected from the group consisting of SEQ ID NOs: 9 to 14.

7. An expression vector comprising the nucleic acid according to claim 5.

8. Cells transformed with the expression vector according to claim 7.

9. A method of producing a Tie2-agonistic antibody or antigen-binding fragment thereof, comprising:
(a) culturing the cells according to claim 8; and
(b) recovering the antibody or antigen-binding fragment thereof from the cultured cells.

10. A composition for preventing or treating an angiogenic disease comprising the antibody or antigen-binding fragment thereof according to any one of claims 1 to 4 as an active ingredient.

11. The composition according to claim 10, wherein the angiogenic disease is selected from the group consisting of cancer, metastasis, diabetic retinopathy, retinopathy of prematurity, corneal graft rejection, macular degeneration, neovascular glaucoma, erythrosis, proliferative retinopathy, psoriasis, hemophilic arthritis, capillary formation of atherosclerotic plaques, keloid, wound granulation, vascular adhesion, rheumatoid arthritis, osteoarthritis, autoimmune diseases, Crohn's disease, restenosis, atherosclerosis, intestinal adhesions, cat scratch disease, ulcer, liver cirrhosis, nephritis, diabetic nephropathy, diabetes mellitus, inflammatory diseases, and neurodegenerative diseases.

12. The composition according to claim 11, wherein the cancer is selected from the group consisting of esophageal cancer, stomach cancer, large intestine cancer, rectal cancer, oral cancer, pharynx cancer, larynx cancer, lung cancer, colon cancer, breast cancer, uterine cervical cancer, endometrial cancer, ovarian cancer, prostate cancer, testicular cancer, bladder cancer, renal cancer, liver cancer, pancreatic cancer, bone cancer, connective tissue cancer, skin cancer, brain cancer, thyroid cancer, leukemia, Hodgkin's lymphoma, lymphoma, and multiple myeloid blood cancer.

13. A composition for co-administration with an additional therapeutic agent for an angiogenic disease, comprising the antibody or antigen-binding fragment thereof according to any one of claims 1 to 4.

14. An antibody polygonal assembly comprising a Tie2-agonistic antibody or antigen-binding fragment thereof and formed with homodimeric Tie2 by binding of the antibody to an Ig3 Fn3 (membrane proximal fibronectin type III) domain of human Tie2.
